(19) Europäisches Patentamt
European Patent Office
Office européen des brevets

(11) Publication number: **0 160 925**
**B1**

(12) # EUROPEAN PATENT SPECIFICATION

(45) Date of publication of patent specification: **18.07.90**

(51) Int. Cl.[5]: **C 07 H 15/00,** A 61 K 31/70

(21) Application number: **85105295.1**

(22) Date of filing: **30.04.85**

(54) **N-substituted neuraminic acid derivatives and process for preparation thereof.**

(30) Priority: **01.05.84 JP 88193/84**

(43) Date of publication of application:
**13.11.85 Bulletin 85/46**

(45) Publication of the grant of the patent:
**18.07.90 Bulletin 90/29**

(84) Designated Contracting States:
**AT BE CH DE FR GB IT LI LU NL SE**

(56) References cited:
**CHEMICAL ABSTRACTS, vol. 64, no. 9. 25th April 1966, columns 12784-12785, Columbus Ohio, US; V.A. DEREVITSKAYA et al.: "Glycopeptides.XVI. Synthesis of the methyl ester of N-glycylmethoxynauramic acid", & KHIM. PRIRODN. SOEDIN., AKAD. NAUK. UZ. SSR 1965(4), 241-4**

(73) Proprietor: **MECT Corporation**
**No. 1780-5, Ohaza Kitano**
**Tokorozawa-Shi Saitama-Ken (JP)**

(72) Inventor: **Shibayama, Shohei**
**5, Noguchi-sou 52-16, Kitanaka 3-chome**
**Tokorozawa-shi Saitama-ken (JP)**
Inventor: **Yoshimura, Shoji**
**3-7-206, Azuma-cho 5-chome**
**Iruma-shi Saitama-ken (JP)**
Inventor: **Ito, Masayoshi**
**27-1-22-303, Fujimidai 1-chome**
**Kunitachi-shi Tokyo (JP)**
Inventor: **Shitori, Yoshiyasu**
**6-12-6-1307, Nishi Shinjuku**
**Shinjuku-ku Tokyo (JP)**
Inventor: **Ogawa, Tomoya**
**3-6-6-3-101, Kita-machi Kichijyouji**
**Musashino-shi Tokyo (JP)**

(74) Representative: **Vossius & Partner**
**Siebertstrasse 4 P.O. Box 86 07 67**
**D-8000 München 86 (DE)**

**EP 0 160 925 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel N-substituted neuraminic acid derivatives having an excellent immunological activity and processes for their preparation.

It is known that n-substituted neuraminic acids are present in many animals and on the cell surface of several bacteria as complexes of sialic acids such as glycoproteins, glycolipids, oligosaccharides or polysaccharides. However, the role of N-substituted neuraminic acids in these complexes has not yet been ascertained.

Recently N-substituted neuraminic acids have become important substances in medicine and pharmaceutics relative to e.g. nerve function, cancer, inflammation, immunity, viral infection, cell differentiation and hormone receptors.

Furthermore many simple derivatives of N-substituted neuraminic acids have been prepared, but no derivative is known for which an excellent immunological activity has been demonstrated.

Chem. Abs. 64: *127* 84F discloses the synthesis of "the methyl ester of N-glycylmethoxy nauramic acid" but does not mention pharmacological activity of the product or its intermediates.

On the other hand, the average span of human life has been extended because of improvements in the medical treatment of malignant tumors of the hematopoietic system of many kinds of cancers, and of collagen diseases. On the other hand, with the great increase in the use of medicines, for example, adrenal cortical hormones or immunosuppressants, a number of undesirable side effects arise together with a decrease in immunological reactivity.

Thus, the technical problem underlying the present invention is to provide novel N-neuraminic acid derivatives having an immunoregulatory effect in which the suppressor T cells are activated, the production of immunoglobulins by B cells is restrained.

Another object of this invention is to provide processes for preparing the novel compounds.

According to the present invention, the novel N-substituted neuraminic acid derivatives have the following general formula (I)

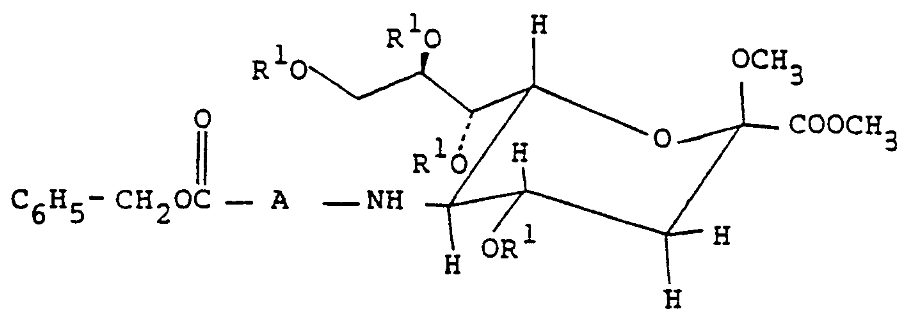

(I)

wherein $R^1$ is a hydrogen atom or an acetyl group; and A is a group having the general formula

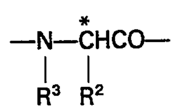

wherein $R^2$ is selected from

$-CH_3$, 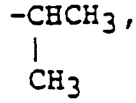 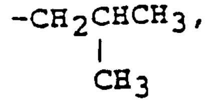

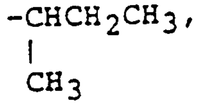 $-CH_2CH_2SCH_3$, $-CH_2C_6H_5$,

$-CH_2OH$, 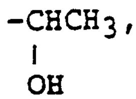 $-CH_2SH$, 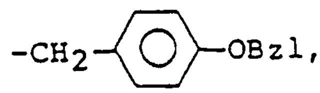

$-CH_2CONH_2$, $-CH_2CH_2CONH_2$. $-(CH_2)_3NH-CBZ$,

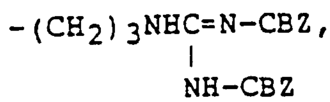 $-CH_2COOBzl$,

$-CH_2CH_2COOBzl$, 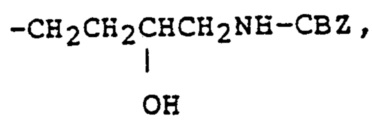

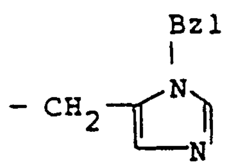 and 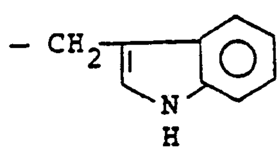

wherein Bzl is $—CH_2C_6H_5$ and CBZ is 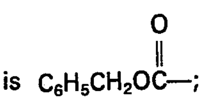;

and $R^3$ is a hydrogen atom or $R^2$ and $R^3$ form the ring selected from

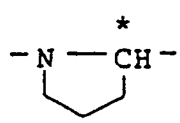 and 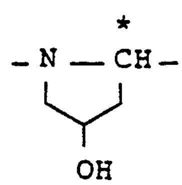

together with the carbon and nitrogen atom to which they are attached. Relative to the steric configuration of the asymmetric carbon atom indicated by an asterisk (*) such as

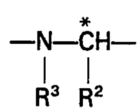

in the formula in the specification, the compounds having said asymmetric carbon atom may occur in the D-form, the L-form or as the racemate.

A compound of the present invention in which $R^1$ in the formula (I) is a hydrogen atom, may be prepared by the following method:

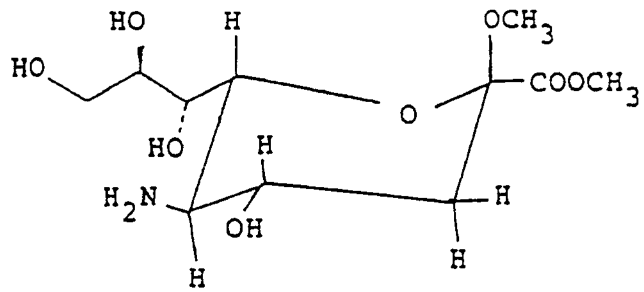

(II)

+

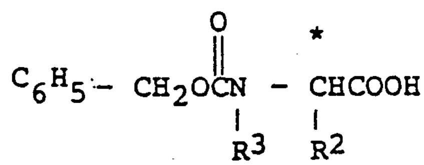

(III)

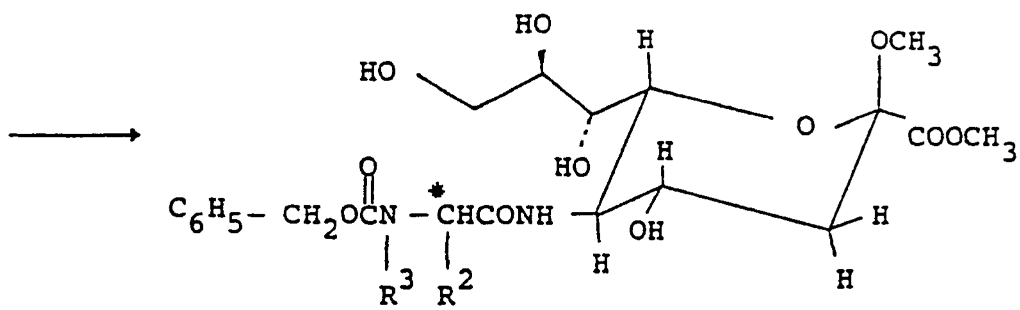

(IV)

For the coupling reaction of the neuraminic acid having the formula (II) with the N-benzyloxycarbonyl amino acid derivative of the formula (III), there are methods in which the compounds are first converted into a reactive derivative and then the coupling reaction is carried out. These methods involve N terminal activation and C terminal activation (a method using an acid chloride, an azide, a mixed acid anhydride, or an active ester).

As a concrete example of the activation, there is a method in which dicyclohexylcarbodiimide (DCC) is added to the mixture of compounds (II) and (III). Solvents such as methylene chloride, acetonitrile, dimethylformamide (DMF), pyridine or tetrahydrofuran may be employed as a solvent in the reaction. The use of DCC, anhydrous pyridine and DMF is preferred.

A general method for inhibiting the racemization in the coupling reaction is known as the Eintopf-method, in which DCC and a N-hydroxy compound are added as reagents for activating the carboxyl group. Examples of N-hydroxy compounds include N-hydroxysuccinic imide (HOSu), 1-hydroxybenzotriazole (HOBt), 3-hydroxy-4-oxo-3,4-dihydro-1,2,3,-benzotriazine (HOOBt) and N-hydroxy-5-norbornene-2,3-dicarboximide (HONB). Tetrahydrofuran or dimethylformamide may be employed as a solvent for the reaction.

The preferred combination of said N-hydroxy compound and the solvent is HONB and dimethylformamide.

A compound of the present invention in which $R^1$ in the formula (I) is an acetyl group may be prepared

by reacting said compound (IV) with acetic anhydride in anhydrous pyridine.

The method for preparing hydrochloride addition salt of compound (II) is described in J.A.C.S., 99, 8273 (1977).

Other novel N-substituted neuraminic acid derivatives have the general formula (V):

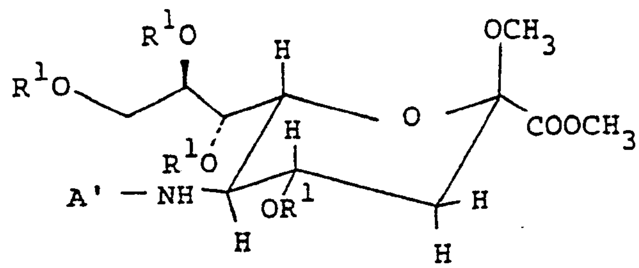

(V)

including inorganic and organic acid addition salts thereof, wherein $R^1$ is a hydogen atom or an acetyl group; and A′ is an amino acid residue the CO-group of which is attached to the —NH group of the above formula.

In the formula (V) A′ preferably has the following formula:

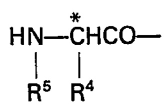

wherein $R^4$ is selected from

$-CH_3$, 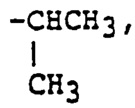 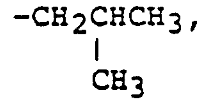

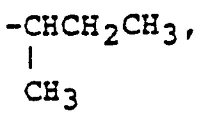 $-CH_2CH_2SCH_3$, $-CH_2C_6H_5$,

$-CH_2OH$, 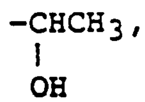 $-CH_2SH$, 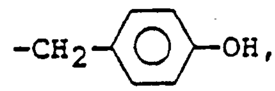

$-CH_2CONH_2$, $-CH_2CH_2CONH_2$, $-(CH_2)_3NH_2$,

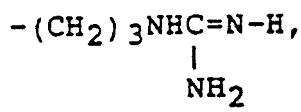 $-CH_2COOH$,

$-CH_2CH_2COOH$, 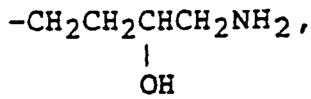

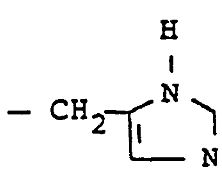 and 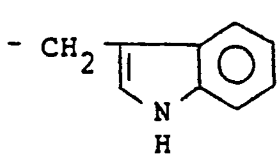

$R^5$ is a hydrogen atom or $R^4$ and $R^5$ form the ring selected from

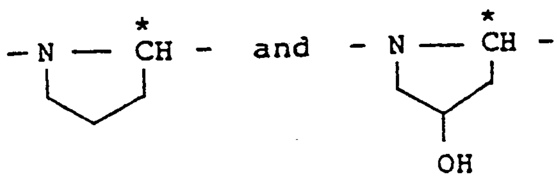

together with the carbon atom and the nitrogen atom to which they are attached.

The compounds of the present invention having the general formula (V) may be prepared by, for example, catalytic reduction of the compounds (I).

Said catalytic reduction may preferably be carried out by decomposing hydrogenation in the presence of palladium on carbon as catalyst an methanol as a solvent. Furthermore, compound (V) may easily be obtained as hydrochlorides by adding ammonium chloride to the reaction mixture.

According to the present invention, the compounds of the formulae (I) and (V) have an excellent immunological activity in adjusting the strength of the immune reaction. The activity capable of adjusting the strength of the immune system can be ascertained by the following method.

The effect of the compounds of the invention on the activation of mouse spleen lymphocyte by Con A:

As T cells are non-specifically activated by Con A, one of the N-substituted neuraminic acid derivatives of the present invention was added to the reaction and then the function thereof was studied. That is, Con A and a compound of the formulae (I) or (V), for example, a compound prepared in the Examples below, were respectively added to spleen lymphocytes (SPC) which were taken from BALB/C mice. The mixture was cultured for about 20 hours at 37°C on a micro plate with 5% $CO_2$ in the atmosphere.

After adding tritium labeled thymidine and after culturing the mixture for about 10 hours SPC were collected and the amount of 3H-thymidine uptake in SPC was determined by using a scintillation counter.

Compounds of the formulae (I) or (V), promote and reinforce the 3H-thymidine uptake and improve the activation of the T-cells by Con A.

The inhibition of the production of immunoglobulins of mouse spleen lymphocytes:

The N-substituted neuraminic acid derivates of the present invention were employed for studying the inhibition of the production of immunoglobulins by measuring the number of plaque-forming cells (PFC).

At first, red blood cells of sheep (SRBC) and one of the compounds as the formula (I) or (V), for example, the compound prepared in the Examples as shown below, was added to SPC and the mixture was cultured at 37°C for 5 days. SRBC and complement were added again to the sensitized SPC thus obtained. The number of PFC was counted after said mixture had been cultured in a Cunningham chamber at 37°C for 3 to 12 hours.

Since a decrease in the number of PFC was observed and the cell viability was the same as in the control, it was ascertained that supression of the production of immunoglobulin was strengthened.

The compounds of the present invention show an excellent activity in the two tests.

Therefore, it is assumed that the production of immunoglobulins was suppressed by activating supressor T cells.

Lowering of the activity of the suppressor T cells has been observed in autoimmune diseases such as the collagen diseases (collagenases). Accordingly, the N-substituted neuraminic acid derivatives of the present invention activating the suppressor T cells are expected to be effective in clinical applications as agents for controlling of the immune system.

The present invention will now be illustrated by referring to the following nonlimitative examples.

Example 1

Preparation of methyl (methyl 5-(N-benzyloxycarbonylglycylamino)-3,5-dideoxy-β-D-glycero-D-galacto-2-nonulopyranosid) onate

3.0 g (0.01 mole) of methyl (methyl 5-amino-3,5-dideoxy-β-D-glycero-D-galacto-2-nonulopyranosid) onate (compound (II)), 2.13 g (0.01 mole) of N-CBZ-glycine (compound (III)) and 3.1 g (0.015 mole) of DCC were dissolved in 350 ml of anhydrous pyridine. The mixture was stirred at room temperature for 48 hours. The resulting suspension was filtrated and the solvent was distilled off. Thereafter, the residue was subjected to silica gel column chromatography and eluted with chloroform/methanol (5:1).

3.92 g of a colorless amorphous substance were obtained (Yield: 80%).

Physical properties of the substances:

Decomposition point: 190—193°C

Elemental analysis: $C_{21}H_{30}N_2O\cdot\frac{13}{10}H_2O$ MW = 509.89

Calculation: C: 49.47 H: 6.44 N: 5.49

Found: C: 49.48 H: 6.63 N: 5.37

$IR\nu_{max}^{KBr}cm^{-1}$:

3,350 (—OH, —NH), 1,740 (—COOMe),

1,700 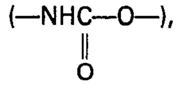,

1,655 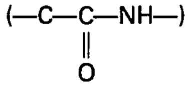

$^1H$-NMR $^{ppm}_{90MHz}$ (DMSO − $d_6$ + $D_2O$, TMS):

1.58 (1H, dd, J=12.2Hz , J=10.8Hz, 3-Hax)

2.27 (1H, dd, J=12.2Hz, J=4.5Hz, 3-Heq)

3.19 (3H, S, 2—OMe)

3.77 (3H, S, —COOMe)

5.08 (2H, S, 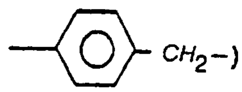

7.39 (5H, S, phenyl-H)

$[\alpha]_D^{20°C}$ − 29.6° (C=1, MeOH)

Example 2

Preparation of methyl (methyl 5-(N-benzyloxycarbonylglycylamino)-3,5-dideoxy-β-D-glycero-D-galacto-2-nonulopyranosid) onate

Example 2 was carried out in the same way as set forth in Example 1 except that 1.15 g (3.9 m mole) of compound (II), 0.82 g (3.9 m mole) of N-CBZ-glycine and 1.21 g (5.85 m mole) of DCC were dissolved in 35 ml of anhydrous DMF. 1.32 g of the title product was obtained (Yield: 70%).

Physical properties of the product:

Decomposition point: 190—193°C

$IR\nu_{max}^{KBr}cm^{-1}$:

3,350 (—OH, —NH), 1,740 (—COOMe),

1,700 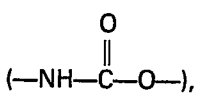,

1,655 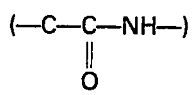

$^1H$-NMR $^{ppm}_{60MHz}$ (DMSO − $d_6$ + $CDCl_3$ + $D_2O$, TMS):

1.59 (1H, m , 3-Hax)

2.34 (1H, m, 3-Heq)

3.24 (3H, S, 2—OMe)

3.77 (3H, S, —COOMe)

5.08 (2H, S, 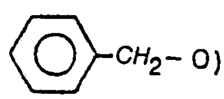

7.35 (5H, S, phenyl-H)

Example 3

Preparation of methyl (methyl 5-(N-benzyloxycarbonylglycylamino)-3,5-dideoxy-β-D-glycero-D-galacto-2-nonulopyranosid) onate

1.0 g (3.39m mole) of (compound (II) and 0.71 g (3.39 m mole) of compound (III) were dissolved in 30 ml

of anhydrous DMF and then 0.31 of N-ethylmorpholine and 0.79 g (4.41 m mole) of HONB (N-hydroxy-5-norbornene-2,3-dicarboximide) were added thereto. Next, 0.91 g (4.41 m mole) of DCC was added. Then the mixture was stirred for 48 hours at room temperature after a 3-hour stirring with simultaneous cooling to a temperature of 0°C. The resulting suspension was filtrated and the solvent was distilled off.

Thereafter, the residue was subjected to silica gel column chromatography and eluted with chloroform/methanol (5:1).

1.35 g of a colorless amorphous substance were obtained (Yield: 82%).

Physical properties of the substance:

Decomposition point: 190—193°C

Elemental analysis: $C_{21}H_{30}N_2O_{11}$ MW = 486.47

Calculation: C: 51.85 H: 6.22 N: 5.76

Found: C: 51.80 H: 6.21 N: 5.64

$IR\nu_{max}^{KBr}cm^{-1}$:

3,350 (—OH, —NH), 1,740 (—COOMe),

1,700 (—NH—$\overset{\overset{O}{\|}}{C}$—O—),

1,655 (—C—$\underset{\underset{O}{\|}}{C}$—NH—)

$^1$H-NMR $^{ppm}_{90MHz}$ (DMSO − $d_6$, TMS):

1.52 (1H, dd, J=12.2Hz , J=10.8Hz, 3-Hax)

2.19 (1H, dd, J=12.2Hz, J=4.5Hz, 3-Heq)

3.18 (3H, S, 2—OMe)

3.71 (3H, S, —COOMe)

5.04 (2H, S, 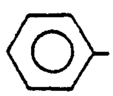 $CH_2$–O–)

7.35 (5H, S, phenyl-H)

Example 4

Preparation of methyl (methyl 5-(N-benzyloxycarbonyl-L-alanylamino)-3,5-dideoxy-β-D-glycero-D-galacto-2-nonulopyranosid) onate

Example 4 was carried out in the same way as set forth in Example 1 except that 1.0 g (3.39 m mole) of compound (II), 0.76 g (3.9 m mole) of N-CBZ-L-alanine and 0.84 g of DCC were dissolved in 100 ml of anhydrous pyridine.

1.18 g of a colorless amorphous substance were obtained (Yield: 70%).

Physical properties of the substance:

Melting point: 74—77°C

Decomposition point: 193—195°C

Elemental analysis: $C_{22}H_{32}N_2O_{11}$ MW = 500.51

Calculation: C: 52.79 H: 6.44 N: 5.60

Found: C: 52.77 H: 6.42 N: 5.58

$IR\nu_{max}^{KBr}cm^{-1}$:

3,350 (—OH, —NH), 1,740 (—COOMe)

1,700 (—NH—$\underset{\underset{O}{\|}}{C}$—O—),

1,660 (—C—$\underset{\underset{O}{\|}}{C}$—NH—)

$^1$H-NMR $^{ppm}_{90MHz}$ (DMSO − $d_6$ + $CDCl_3$ + $D_2O$, TMS):

1.40 (3H, d, J=7.07Hz >CH—$CH_3$)

1.68 (1H, dd, J=13.5Hz , J=10.8Hz, 3-Hax)

2.38 (1H, dd, J=13.5Hz, J=4.9Hz, 3-Heq)
3.26 (3H, S, 2—$OCH_3$)
3.73 (3H, S, —$COOCH_3$)

5.05 (2H, S, 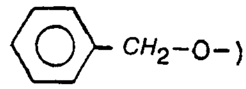

7.29 (5H, S, phenyl-H)
$[\alpha]_D^{20°C}$ − 29.9° (C=1, MeoH)

Example 5

Preparation of methyl (methyl 5-(N-benzyloxycarbonyl-L-alanylamino)-3,5-dideoxy-β-D-glycero-D-galacto-2-nonulopyranosid) onate

Example 5 was carried out in the same way as set forth in Example 3 except that 0.76 g (3.39 m mole) of N-CBZ-L-alanine was used instead of N-CBZ-glycine. 1.40 g of the title product was obtained (Yield: 83%).

Physical properties of the product:
Decomposition point: 198—201°C

Example 6

Preparation of methyl (methyl 5-(N-benzyloxycarbonyl-L-leucylamino)-3,5-dideoxy-β-D-glycero-D-galacto-2-nonulopyranosid) onate

Example 6 was carried out in the same way as set forth in Example 4 except that 0.9 g (3.39 m mole) of N-CBZ-L-leucine was used instead of N-CBZ-L-alanine and that the solvent used for the elution was chloroform/methanol (10:1).

1.38 g of the title product was obtained (Yield: 75%).

Physical properties of the product:
Melting point: 76—81°C
Decomposition point: 156—158°C
Elemental analysis: $C_{25}H_{38}N_2O_{11}\cdot\frac{1}{5}H_2O$ MW = 546.20

| | | | |
|---|---|---|---|
| Calculation: | C: 54.98 | H: 7.09 | N: 5.13 |
| Found: | C: 54.97 | H: 6.95 | N: 5.05 |

$IR\nu_{max}^{KBr}cm^{-1}$:
3,375 (—OH, —NH—), 1,740 (—COOMe)

1,700 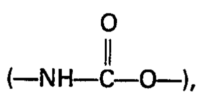,

1,655 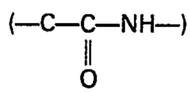

$^1H$-NMR $^{ppm}_{90MHz}$ (DMSO − $d_6$ + $CDCl_3$ + $D_2O$, TMS):
0.94 (6H, d, J=4.9Hz,

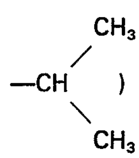

2.37 (1H, dd, J=13.5Hz, J=5.4Hz, 3-Heq)
3.27 (3H, S, 2—OMe)
3.78 (3H, S, —$COOCH_3$)

5.09 (2H, S, 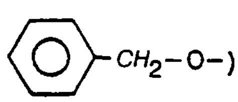

7.34 (5H, S, phenyl-H)
$[\alpha]_D^{20°C}$ − 29.3° (C=1, MeoH)

The compound as set forth in Example 6 was also prepared in the same way as set forth in Example 5

except that 5.39 g of N-CBZ-L-leucine was used instead of N-CBZ-L-alanine, and that 6.0 g of the compound II, 1.86 ml of N-ethylmorpholine and 4.74 g of HONB were used. 7.7 g of the product were obtained (Yield 70%). The physical properties of the product were the same as shown above.

Example 7

Preparation of methyl (methyl 5-(N-benzyloxycarbonyl-L-phenylalanylamino)-3,5-dideoxy-β-D-glycero-D-galacto-2-nonulopyranosid) onate

Example 7 was carried out in the same way as set forth in Example 4 except that 1.015 g (3.39 m mole) of N-CBZ-L-phenylalanine was used instead of N-CBZ-L-alanine).

1.52 g of the product were obtained (Yield: 78%).

Physical properties of the product:

Melting point: 75—80°C

Decomposition point: 168—170°C

Elemental analysis: $C_{28}H_{36}N_2O_{11}$ MW = 576.60

| | | | |
|---|---|---|---|
| Calculation: | C: 58.33 | H: 6.29 | N: 4.86 |
| Found: | C: 58.32 | H: 6.17 | N: 4.83 |

$IR\nu_{max}^{KBr}cm^{-1}$:

3,375 (—OH, —NH—), 1,735 (—COOMe)

1,700 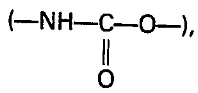,

1,655 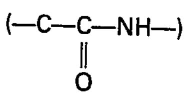

$^1H$-NMR $\delta_{90MHz}^{ppm}$ (DMSO − $d_6$ + $D_2O$, TMS):

1.55 (1H, dd, J=12.6Hz , J=10.8Hz, 3-Hax)

2.22 (1H, dd, J=12.6Hz, J=4.05Hz, 3-Heq)

3.21 (3H, S, 2—$OCH_3$)

3.74 (3H, S, —$COOCH_3$)

4.96 (2H, S, 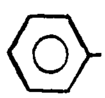 $CH_2$–O–)

$[\alpha]_D^{20°C}$ − 22.8° (C=1, MeOH)

The compound as set forth in Example 7 was also prepared in the same way as set forth in Example 5 except that 6.08 of N-CBZ-L-phenylalanine was used instead of N-CBZ-L-alanine, and that 6.0 g of the compound II, 1.86 ml of N-ethylmorpholine and 4.74 g of HONB were used. 8.4 g of the product were obtained (Yield 72%). The physical properties of the product were the same as shown above.

Example 8

Preparation of methyl (methyl 5-(N-benzyloxycarbonyl-L-serylamino)-3,5-dideoxy-β-D-glycero-D-galacto-2-nonulopyranosid) onate

Example 8 was carried out in the same way as set forth in Example 4 except that 0.81 g (3.39 m mole) of N-CBZ-L-serine was used instead of N-CBZ-L-alanine.

1.33 g of the product were obtained (Yield: 76%).

Physical properties of the product:
Melting point: 71—76°C
Decomposition point: 147—154°C
Elemental analysis: $C_{22}H_{32}N_2O_{12}$ MW = 516.51
Calculation: C: 51.16 H: 6.24 N: 5.42
Found: C: 51.13 H: 6.17 N: 5.46
$IR\nu_{max}^{KBr}cm^{-1}$:
3,375 (—OH, —NH—), 1,740 (—COOMe)

1,700 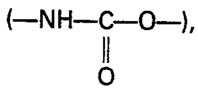,

1,655 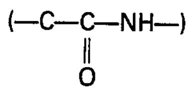

$^1H$-NMR $^{ppm}_{90MHz}$ (DMSO − $d_6$ + $D_2O$, TMS):
1.51 (1H, dd, J=12.6Hz , J=11.25Hz, 3-Hax)
2.21 (1H, dd, J=12.6Hz, J=5.0Hz, 3-Heq)
3.17 (3H, S, 2—$OCH_3$)
3.72 (3H, S, —$COOCH_3$)

5.04 (2H, S, 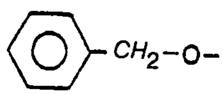)

7.37 (5H, S, phenyl-H)

$[\alpha]_D^{20°C}$ − 22.63° (C=1, MeOH)

The compound as set forth in Example 8 was also prepared in the same way as set forth in Example 5 except that 4.86 of N-CBZ-L-serine was used instead of N-CBZ-L-alanine, and that 6.0 g of the compound II, 2.42 ml of N-ethylmorpholine and 4.73 g of HONB were used. 7.4 g of the product were obtained (Yield 70%). The physical properties of the product were the same as shown above.

## Example 9

Preparation of methyl (methyl 5-(N-benzyloxycarbonyl-L-valylamino)-3,5-dideoxy-β-D-glycero-D-galacto-2-nonulopyranosid) onate

Example 9 was carried out by the same procedures as set forth in Example 4 except that 0.85 g (3.39 m mole) of N-CBZ-L-valine was used instead of N-CBZ-L-alanine and that chloroform/methanol (10:1) was used as solvent for elution 1.43 g of the product were obtained (Yield: 80%).

Physical properties of the product:
Melting point: 78—83°C
Decomposition point: 153—154°C
Elemental analysis: $C_{24}H_{36}N_2O_{11}\cdot\frac{2}{5}H_2O$ MW = 535.77
Calculation: C: 53.80 H: 6.92 N: 5.23
Found: C: 53.83 H: 6.81 N: 5.18
$IR\nu_{max}^{KBr}cm^{-1}$:
3,375 (—OH, —NH), 1,730 (—$COOCH_3$)

1,700 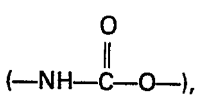,

1,650 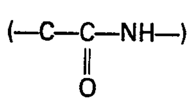

$^1$H-NMR $^{ppm}_{90MHz}$ (DMSO − $d_6$ + $D_2O$, TMS):

0.87; 0.90 (6H, d, J=6.6Hz,

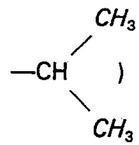

)

1.55 (1H, dd, J=12.7Hz , J=10.9Hz, 3-Hax)

1.73—2.10 (1H, m,

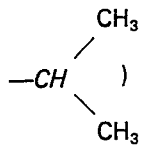

)

2.23 (1H, dd, J=12.7Hz, J=4.4Hz, 3-Heq)

3.19 (3H, S, 2—$OCH_3$)

3.74 (3H, S, —$COOCH_3$)

3.19—3.91 (8H, m, sialy-H,

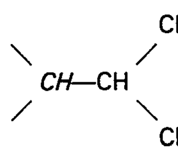

)

5.05 (2H, S, $C_6H_5CH_2O$—)

7.37 (5H, S, $C_6H_5$—)

$[\alpha]_D^{20°C}$ − 26.2° (C=1, MeOH)

The compound as set forth in Example 9 was also prepared in the same way as set forth in Example 5 except that 5.62 of N-CBZ-L-valine was used instead of N-CBZ-L-alanine, and that 6.0 g of the compound II, 2.35 ml of N-ethylmorpholine and 4.73 g of HONB were used. 78.4 g of the product were obtained (Yield 73%). The physical properties of the product were the same as shown above.

Example 10

Preparation of methyl (methyl 5-(N-benzyloxycarbonyl-L-methionylamino)-3,5-dideoxy-β-D-glycero-D-galacto-2-nonulopyranosid) onate

Example 10 was carried out in the same way as set forth in Example 4 except that 0.96 g (3.39 m mole) of N-CBZ-L-methionine was used instead of N-CBZ-L-alanine and chloroform/methanol (10:1) was used as solvent for elution 1.48 g of the product were obtained (Yield 78%).

Physical properties of the product:

Melting point: 67—72°C

Decomposition point: 170—177°C

Elemental analysis: $C_{24}H_{36}N_2O_{11}S$ MW = 560.63

| | | | |
|---|---|---|---|
| Calculation: | C: 51.42 | H: 6.47 | N: 5.00 |
| Found: | C: 51.28 | H: 6.37 | N: 4.97 |

IR$\nu^{KBr}_{max}$cm$^{-1}$:

3,350 (—OH, —NH), 1,730 (—$COOCH_3$),

1,700 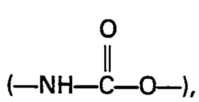,

1,650 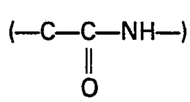

$^1$H-NMR $^{ppm}_{90MHz}$ (DMSO − $d_6$ + $D_2O$, TMS):
1.56 (1H, dd, J=12.7Hz , J=11.0Hz, 3-Hax)
1.71—2.00 (2H, m, $CH_3SCH_2$*$CH_2$*—)
2.04 (3H, S, *$CH_3$*—S—)
2.25 (1H, dd, J=12.7Hz, J=4.0Hz, 3-Heq)
2.35—2.62 (2H, m, $CH_3S$*$CH_2$*$CH_2$—)
3.19 (3H, S, 2—$OCH_3$)
3.75 (3H, S, —$COOCH_3$)
3.19—3.85 (8H, m, sialyl-H, $CH_3SCH_2CH_2$*CH*<
5.05 (2H, S, $C_6H_5$*$CH_2$*O—)
7.37 (5H, S, $C_6H_5$—)

$[\alpha]_D^{20°C}$ − 27.5° (C=1, MeOH)

The compound as set forth in Example 10 was also prepared in the same way as set forth in Example 5 except that 5.76 of N-CBZ-L-methionine was used instead of N-CBZ-L-alanine, and that 6.0 g of the compound II, 2.35 ml of N-ethylmorpholine and 4.73 g of HONB were used. 8.1 g of the product was obtained (Yield 71%). The physical properties of the product were the same as shown above.

## Example 11

Preparation of methyl (methyl 5-(glycylamino)-3,5-dideoxy-β-D-glycero-D-galacto-2-nonulopyranosid) onate

300 mg (1.34 m mole) of methyl (methyl 5-(N-benzyloxycarbonylglycylamino-3,5-dideoxy-β-D-glycero-D-galacto-2-nonulopyranosid) onate was dissolved into anhydrous methanol and thereafter 301 mg (0.62 m mole) of palladium on carbon (10%) were added thereto. Then the mixture was stirred under hydrogen gas at room temperature for 16 hours. The resulting reaction suspension was filtrated and the solvent was distilled off. 210 mg of a colorless amorphous substance were obtained (Yield 97%).

Physical properties of the product:
Decomposition point: 126—130°C
$IR\nu^{KBr}_{max}cm^{-1}$:
1,740 (—$COOCH_3$)
$^1$H-NMR $^{ppm}_{60MHz}$ ($CD_3OD$, TMS):
1.61 (1H, m, 3-Hax)
2.40 (1H, m, 3-Heq)
3.27 (3H, S, 2—$OCH_3$)
3.81 (3H, S, —$COOCH_3$)
3.41—4.36 (9H, m sialyl-H, $NH_2$*$CH_2$*CO—)

## Example 12

Preparation of the hydrochloride of methyl (methyl 5-(glycylamino)-3,5-dideoxy-β-D-glycero-D-galacto-2-nonulopyranosid) onate

500 mg (10.28 m mole) of methyl (methyl 5-(N-benzyloxycarbonylglycylamino)-3,5-deoxy-β-D-glycero-D-galacto-2-nonulopyranosid) onate were dissolved into anhydrous methanol and thereafter 428 mg (0.4 m mole) of palladium on carbon (10%) and 200 mg (3.67 m mole) of $NH_4Cl$ were added thereto. Then the mixture was stirred under hydrogen gas at room temperature for 20 hours. The resulting reaction suspension was filtrated and the solvent was distilled off. 380 mg of a colorless amorphous substance were obtained (Yield 95%).

Physical properties of the product:
Decomposition point: 145—150°C
$IR\nu^{KBr}_{max}cm^{-1}$:
1,740 (—$COOCH_3$),
$^1$H-NMR $^{ppm}_{60MHz}$ ($CD_3OD$, TMS):
1.62 (1H, m, 3-Hax)
2.36 (1H, m, 3-Heq)
3.27 (3H, S, 2—$OCH_3$)
3.83 (3H, S, —$COOCH_3$)
3.40—4.20 (9H, m sialyl-H, $NH_2$*$CH_2$*CO—)

## Example 13

Preparation of the methyl (methyl 5-(N-benzyloxycarbonylglycylamino)-3,5-dideoxy-4,7,8,9-tetra-O-acetyl-β-D-glycero-D-galacto-2-nonulopyranosid) onate

0.15 g (0.31 m mole) of methyl (methyl 5-(N-benzyloxycarbonylglycylamino)-3,5-dideoxy-β-D-glycero-D-galacto-2-nonulopyranosid) onate were dissolved in 3 ml of anhydrous pyridine and thereafter 3 ml of acetic anhydride were added. Then the mixture was stirred at room temperature for two hours. The

resulting reaction mixture was filtrated and the solvent was distilled off. Thereafter the residue was subjected to silica gel chromatography and eluted with chloroform/methanol (30:1). 0.17 g of a colorless amorphous substance were obtained (Yield 85%).

Physical properties of the substance:
Melting point: 70—74°C
Decomposition point: 130—160°C
$IR\nu_{max}^{KBr} cm^{-1}$:
3,370 (—NH), 1,740 (—$COOCH_3$)

$^1$H-NMR $^{ppm}_{90MHz}$ ($CDCl_3$, TMS):
1.57—2.14 (13H, m, $CH_3CO$—, 3-Hax)
2.47 (1H, dd, J=13.0Hz, J=5.27Hz, 3-Heq)
3.23 (3H, S, 2—$OCH_3$)
3.80 (3H, S, —$COOCH_3$)

5.14 (2H, S, 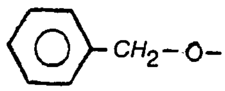 )

3.61—5.50 (9H, m, —NH$CH_2$—CO—, sialyl-H)
5.62·6.62 (2H, broad S, —NH—)
7.35 (5H, S, phenyl-H)

Example 14

Preparation of methyl (methyl 5-(N-benzyloxycarbonyl-D-alanylamino)-3,5-dideoxy-β-D-glycero-D-galacto-2-nonulopyranosid) onate

Example 14 was carried out in the same way as set forth in Example 5 except that 0.76 g of N-CBZ-D-alanine was used instead of N-CBZ-L-alanine, and that 1.0 g of compound II, 0.36 ml of N-ethylmorpholine and 0.79 g of HONB were used. 1.2 g of the product were obtained (Yield 70%).

Physical properties of the product:
Decomposition : 209—213°C
Elemental analysis: $C_{12}H_{32}N_2O_{11}$ MW = 500.51
Calculation: C: 52.79 H: 6.44 N: 5.60
Found: C: 52.97 H: 6.40 N: 5.73
$IR\nu_{max}^{KBr} cm^{-1}$:
3,400 (—OH, —NH), 1,740 (—COO $CH_3$),

1,700 (—NH C—O—),
‖
O

$^1$H-NMR $^{ppm}_{90MHz}$ (DMSO-$d_6$ + $D_2O$, TMS):
1.26 (3H, d, J=7.07Hz, >CH—$CH_3$)
1.52 (1H, dd, J=13.0Hz, J=10.8Hz, 3-Hax)
2.21 (1H, dd, J=13.0Hz, H=4.5Hz, 3-Heq)
3.18 (3H, S, 2—$OCH_3$)
3.74 (3H, S, —$COOCH_3$)
5.04 (2H, S, Ø—$CH_2$—O—)
7.37 (5H, S, phenyl-H)
$[\alpha]_D^{22.5°C}$ − 12.91° (C=1, MeOH)

Example 15

Preparation of methyl (methyl 5-(N-benzyloxycarbonyl-DL-alanylamino)-3,5-dideoxy-β-D-glycero-D-galacto-2-nonulopyranosid) onate

Example 15 was carried out in the same way as set forth in Example 5 except that 0.76 g of N-CBZ-DL-alanine was used instead of N-CBZ-L-alanine, and that 1.0 g of compound II, 0.36 ml of N-ethylmorpholine and 0.79 g of HONB were used. 1.25 g of the product were obtained (Yield 74%).

Physical properties of the product:

Decomposition point: 192—195°C

Elemental analysis: $C_{12}H_{32}N_2O_{11}$ MW = 500.51

Calculation: C: 52.79 H: 6.44 N: 5.60

Found: C: 52.88 H: 6.30 N: 5.65

$^{1}$H-NMR $^{ppm}_{400MHz}$ (DMSO-$d_6$ + $D_2O$, TMS):

1.218; 1.223 (6H, d, J=7.10Hz, >CH—$CH_3$)

1.503 (1H, m 3-Hax)

2.161 (1H, m, 3-Heq)

3.168; 3.176 (3H, S, 2—$OCH_3$)

4.045; 4.142 (1H, m, >*CH*—$CH_3$)

IR$\nu^{KBr}_{max}$$cm^{-1}$:

3,400 (—OH, —NH), 1,740 (—$COOCH_3$),

1,700 (—NHC—O—),
||
O

1,660 (—C—C—NH—)
||
O

Example 16

Preparation of methyl (methyl 5-(N-benzyloxycarbonyl-D-valylamino)-3,5-dideoxy-β-D-glycero-D-galacto-2-nonulopyranosid) onate

Example 16 was carried out in the same way as set forth in Example 5 except that 0.43 g of N-CBZ-D-valine were used instead of N-CBZ-L-alanine, and that 0.5 g of compound II, 0.18 ml of N-ethylmorpholine and 0.40 g of HONB were used. 627 mg of the product were obtained (Yield 70%).

Physical properties of the product:

Decomposition point: 229—231°C

Elemental analysis: $C_{24}H_{36}N_2O_{11}$ MW = 528.56

Calculation: C: 54.54 H: 6.87 N: 5.30

Found: C: 54.61 H: 6.72 N: 5.44

IR$\nu^{KBr}_{max}$$cm^{-1}$:

3,400 (—OH, —NH), 1,740 (—$COOCH_3$),

1,700 (—NH—C—O—),
||
O

$[\alpha]_D^{25.6°C}$ − 33.8° (C=1, DMF)

Example 17

Preparation of methyl (methyl 5-L-alanylamino-3,5-dideoxy-β-D-glycero-D-galacto-2-nonulopyranosid) onate

Example 17 was carried out in the same way as set forth in Example 11 except that 1.5 g of the colorless amorphous substance prepared in Example 4 were used as a starting material. 0.77 g of the product were obtained (Yield 70%).

Physical properties of the product obtained:

Decomposition point: 164—170°C

IR$\nu^{KBr}_{max}$$cm^{-1}$:

3,300 (—$NH_2$, —OH), 1,740 (—$COOCH_3$)

$^{1}$H-NMR $^{ppm}_{90MHz}$ (DMSO−$d_6$ + $D_2O$, TMS):

1.19 (3H, d, J=6.8Hz, —$CH_3$)

3.17 (3H, S, 2—$OCH_3$)

3.73 (3H, S, —$COOCH_3$)

Example 18

Preparation of methyl (methyl 5-L-leucylamino-3,5-dideoxy-β-D-glycero-D-galacto-2-nonulopyranosid) onate

Example 18 was carried out in the same way as set forth in Example 11 except that 1.5 g of the product prepared in Example 6 were used as a starting material. 0.95 g of the product were obtained (Yield 82%).

Physical properties of the product obtained:
Decomposition point: 196—199°C
$IRv_{max}^{KBr}cm^{-1}$:
3,400—3,200 (—$NH_2$, —OH), 1,740 (—$COOCH_3$)
$^{1}$H-NMR $^{ppm}_{90MHz}$ (DMSO−$d_6$, TMS):
0.89 (6H, d, J=6.18Hz,

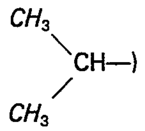

3.18 (3H, S, 2—$OCH_3$)
3.71 (3H, S, —$COOCH_3$)

Example 19

Preparation of methyl (methyl 5-L-phenylalanylamino-3,5-dideoxy-β-D-glycero-D-galacto-2-nonulopyranosid) onate

Example 19 was carried out in the same way as set forth in Example 11 except that 1.5 g of the product prepared in Example 7 were used as a starting material. 0.91 g of the product were obtained (Yield 79%).

Physical properties of the product obtained:
Decomposition point: 181—187°C
$IRv_{max}^{KBr}cm^{-1}$:
3,350 (—OH, —$NH_2$), 1,740 (—$COOCH_3$)
$^{1}$H-NMR $^{ppm}_{90MHz}$ (DMSO−$d_6$, TMS):
3.18 (3H, S, 2—$OCH_3$)
3.71 (3H, S, —$COOCH_3$)

Example 20

Preparation of methyl (methyl 5-L-serylamino-3,5-dideoxy-β-D-glycero-D-galacto-2-nonulopyranosid) onate

Example 20 was carried out in the same way as set forth in Example 11 except that 1.5 g of the product prepared in Example 8 were used as a starting material. 0.91 g of the product were obtained (Yield 82%)

Physical properties of the product obtained:
Decomposition point: 118—124°C
$IRv_{max}^{KBr}cm^{-1}$:
3,350 (—OH, —$NH_2$), 1,740 (—$COOCH_3$)
$^{1}$H-NMR $^{ppm}_{90MHz}$ (DMSO−$d_6$ + $D_2O$, TMS):
3.17 (3H, S, 2—$OCH_3$)
3.73 (3H, S, —$COOCH_3$)

Example 21

Preparation of methyl (methyl 5-L-varylamino-3,5-dideoxy-β-D-glycero-D-galacto-2-nonulopyranosid) onate

Example 21 was carried out in the same way as set forth in Example 11 except that 1.5 g of the product prepared in Example 9 were used as a starting material. 0.93 g of the product were obtained (Yield 83%)

Physical properties of the product obtained:
Decomposition point: 112—118°C
$IRv_{max}^{KBr}cm^{-1}$:
3,300—3,400 (—OH, —$NH_2$), 1,740 (—$COOCH_3$)
$^{1}$H-NMR $^{ppm}_{90MHz}$ (DMSO−$d_6$, TMS):
0.897 (6H, d, J=6.2Hz,

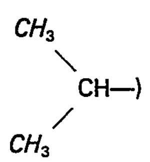

3.180 (3H, S, 2—$OCH_3$)
3.710 (3H, S, —$COOCH_3$)

**Claims for the Contracting States: BE CH DE FR GB IT LI LU NL SE**

1. N-substituted neuraminic acid derivatives having the general formula:

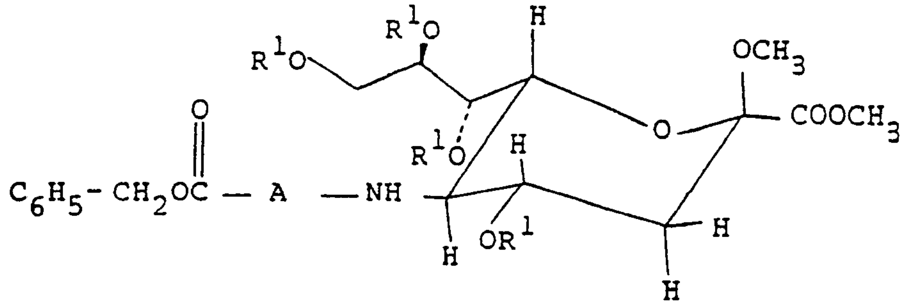

(I)

wherein $R^1$ is a hydrogen atom or an acetyl group: and A is a group having the general formula:

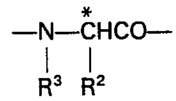

wherein $R^2$ is selected from

$-CH_3$, 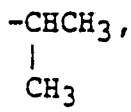 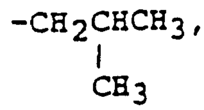

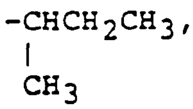 $-CH_2CH_2SCH_3$, $-CH_2C_6H_5$,

$-CH_2OH$, 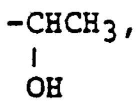 $-CH_2SH$, 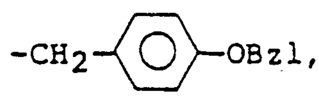

$-CH_2CONH_2$, $-CH_2CH_2CONH_2$. $-(CH_2)_3NH$-CBZ,

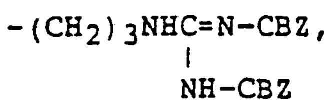 $-CH_2COO$Bzl,

$-CH_2CH_2COO$Bzl, 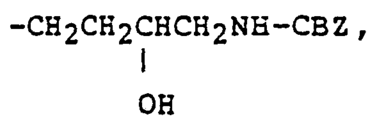

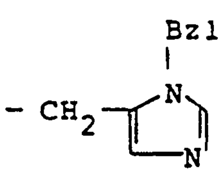 and 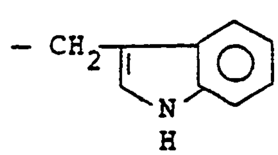

wherein BZl is $-CH_2C_6H_5$ and CBZ is

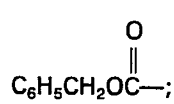

and $R^3$ is a hydrogen atom or $R^2$ and $R^3$ form the ring selected from

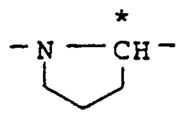

and

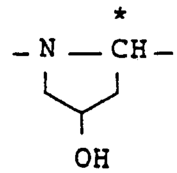

together with the carbon and nitrogen atom to which they are attached.

2. A process for preparing N-substituted neuraminic acid derivatives having the general formula:

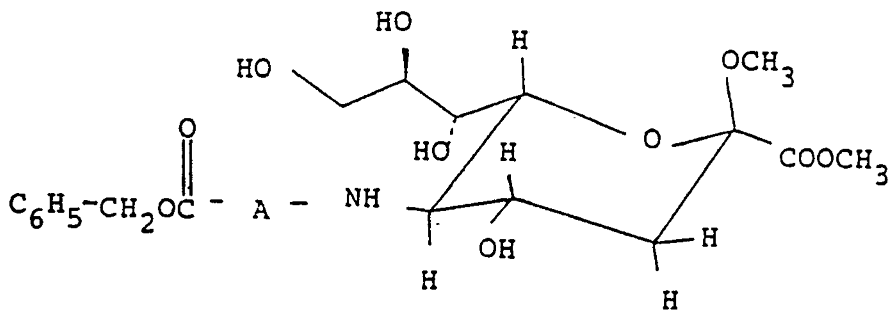

(I-a)

wherein A has the meaning as given in claim 1, which comprises reacting an N-benzyloxycarbonyl amino acid derivative having the general formula:

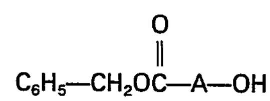

with a neuraminic acid derivative having the formula:

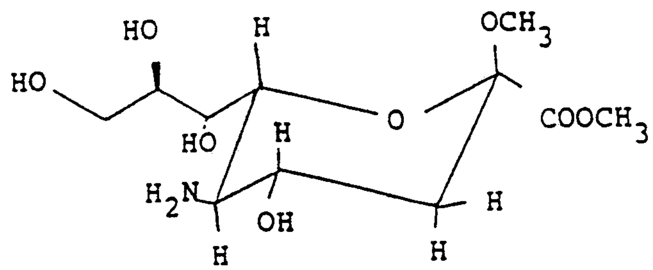

(II)

3. A process as claimed in claim 2 wherein the reaction is carried out in a solvent selected from the group consisting of anhydrous pyridine and dimethylformamide in the presence of dicyclohexylcarbodiimide.

4. A process as claimed in claim 2 wherein the N-hydroxy compound is added to dimethylformamide in the presence of dicyclohexylcarbodiimide.

5. A process for preparing N-substituted neuraminic acid derivatives having the general formula:

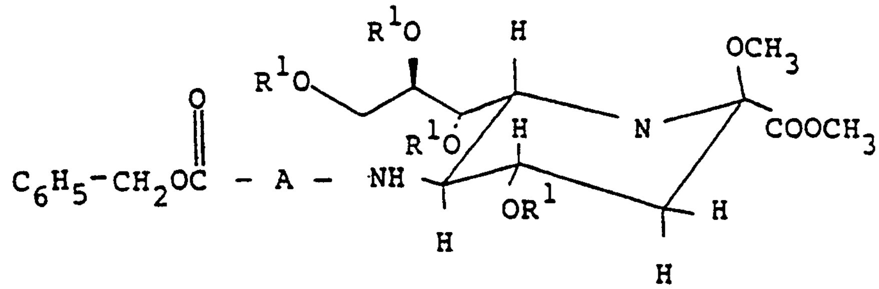

(I-b)

wherein A has the meaning as given in claim 1, and $R^1$ is an acetyl group, which comprises reacting an N-substituted neuraminic acid derivative having the general formula:

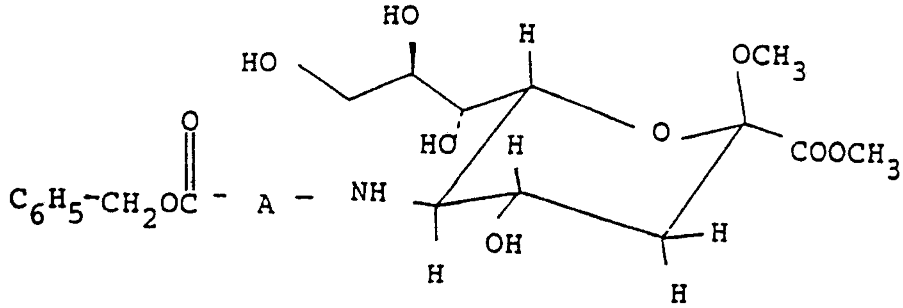

(I-a)

with acetic anhydride in anhydrous pyridine.

6. N-substituted neuraminic acid derivatives having the general formula:

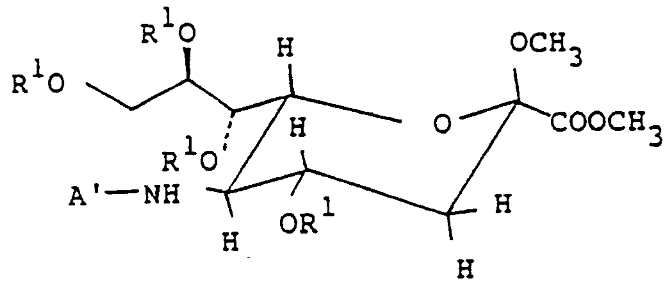

(II-a)

and inorganic and organic acid addition salts thereof, wherein $R^1$ is a hydrogen atom or an acetyl group and A′ is an amino acid residue having the general formula:

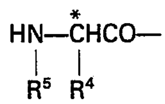

wherein $R^4$ is selected from

$-CH_3$, 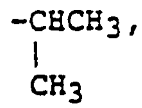, 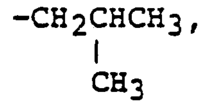,

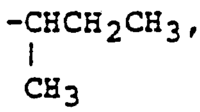, $-CH_2CH_2SCH_3$, $-CH_2C_6H_5$,

$-CH_2OH$, 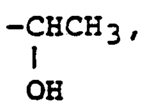, $-CH_2SH$, 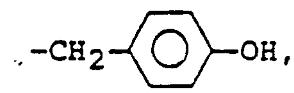,

$-CH_2CONH_2$, $-CH_2CH_2CONH_2$, $-(CH_2)_3NH_2$,

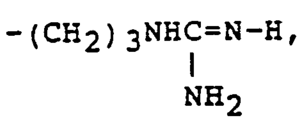, $-CH_2COOH$,

$-CH_2CH_2COOH$, 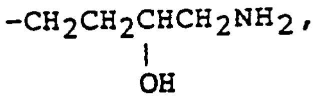,

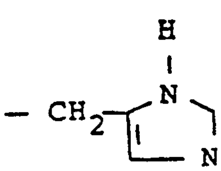 and 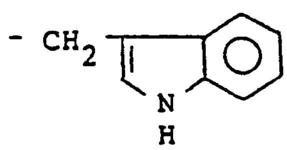

wherein $R^5$ is a hydrogen atom or $R^4$ and $R^5$ form the ring selected from

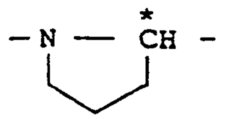

and

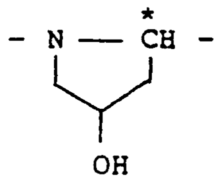

together with the carbon atom and the nitrogen atom to which they are attached.

7. A process for preparing N-substituted neuraminic acid derivatives having the general formula:

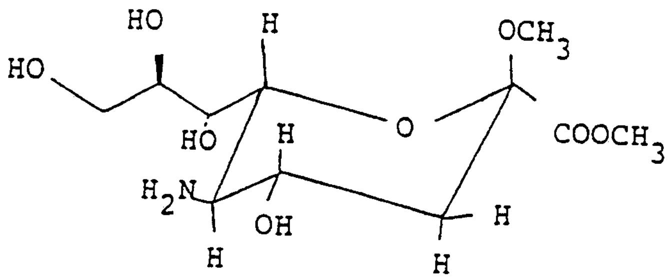

(II)

wherein $R^1$ and A' have the meaning as given in claim 6, and inorganic and organic acid addition salts thereof, which comprises subjecting an N-substituted neuraminic acid derivative of the general formula:

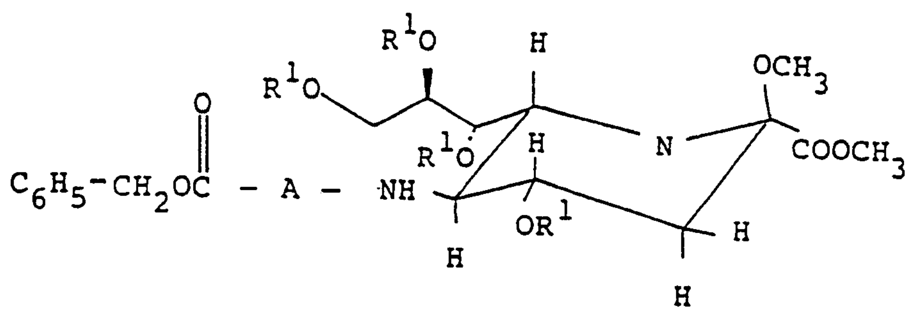

(I-b)

wherein A has the meaning as given in claim 1, to catalytic reduction.

8. A process as claimed in claim 7 wherein A' is an amino acid residue having the general formula:

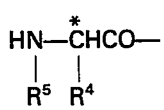

wherein $R^4$ is selected from

$-CH_3$, $-CHCH_3$, (with $CH_3$ below the CH) $-CH_2CHCH_3$, (with $CH_3$ below the CH)

$-CHCH_2CH_3$, (with $CH_3$ below the CH) $-CH_2CH_2SCH_3$, $-CH_2C_6H_5$,

$-CH_2OH$, $-CHCH_3$, (with OH below the CH) $-CH_2SH$, 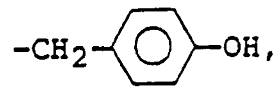

$-CH_2CONH_2$, $-CH_2CH_2CONH_2$, $-(CH_2)_3NH_2$,

$-(CH_2)_3NHC{=}N{-}H$, (with $NH_2$ below the C) $-CH_2COOH$,

$-CH_2CH_2COOH$, $-CH_2CH_2CHCH_2NH_2$, (with OH below the CH)

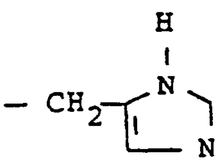 and 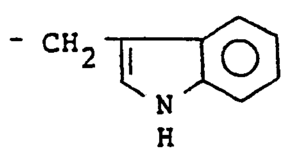

$R^5$ is hydrogen or $R^4$ and $R^5$ form the ring selected from

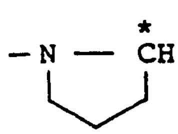 and 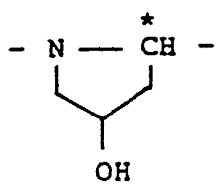

together with the carbon and nitrogen atom to which they are attached.

9. A process as set forth in claim 7 wherein the catalytic reduction is carried out with hydrogen in the presence of a palladium-on-carbon catalyst.

10. Pharmaceutical compositions having immunoregulating effects comprising a compound according to any one of claims 1 and 6.

**Claims for the Contracting State: AT**

1. A process for preparing N-substituted neuraminic acid derivatives having the general formula:

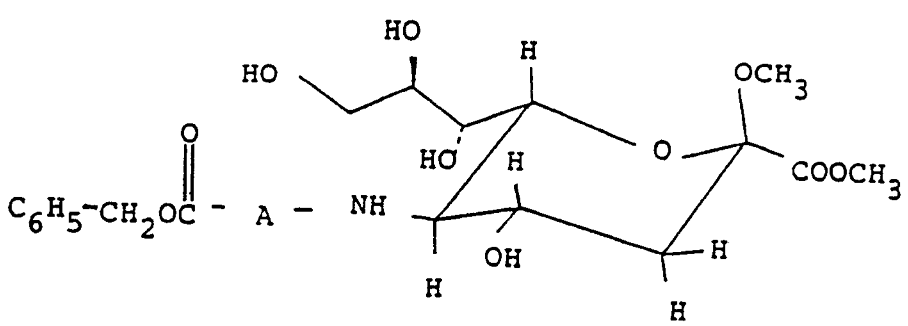

(I-a)

wherein A is a group having the following general formula:

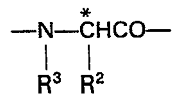

wherein $R^2$ is selected from

$-CH_3$, 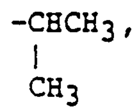 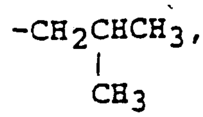

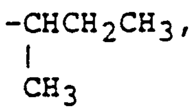 $-CH_2CH_2SCH_3$, $-CH_2C_6H_5$,

$-CH_2OH$, 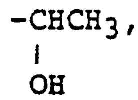 $-CH_2SH$, 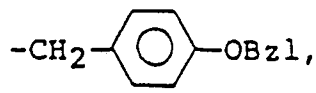

$-CH_2CONH_2$, $-CH_2CH_2CONH_2$. $-(CH_2)_3NH-CBZ$,

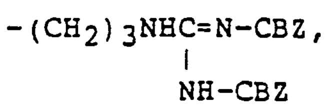 $-CH_2COOBzl$,

$-CH_2CH_2COOBzl$, 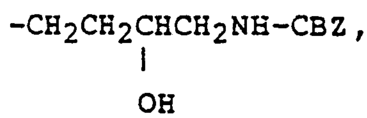

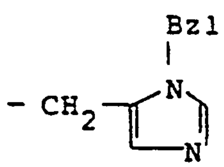 and 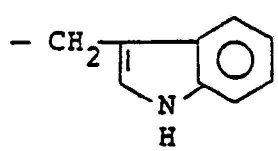

wherein BZl is $—CH_2C_6H_5$ and CBZ is

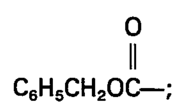

and $R^3$ is a hydrogen atom or $R^2$ and $R^3$ form the ring selected from

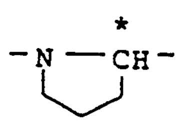 and 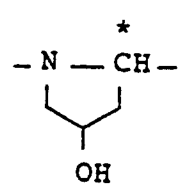

together with the carbon and nitrogen atom to which they are attached, which comprises reacting an N-benzyloxycarbonyl amino acid derivative having the general formula:

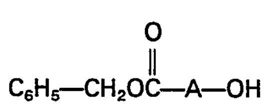

with a neuraminic acid derivative having the formula:

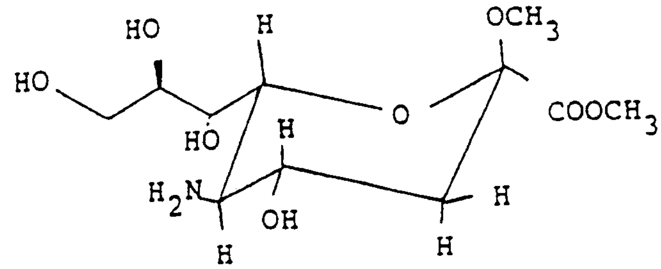

(II)

2. A process as claimed in claim 1 wherein the reaction is carried out in a solvent selected from the group consisting of anhydrous pyridine and dimethylformamide in the presence of dicyclohexylcarbodiimide.

3. A process as claimed in claim 1 wherein the N-hydroxy compound is added to dimethylformamide in the presence of dicyclohexylcarbodiimide.

4. A process for preparing N-substituted neuraminic acid derivatives having the general formula:

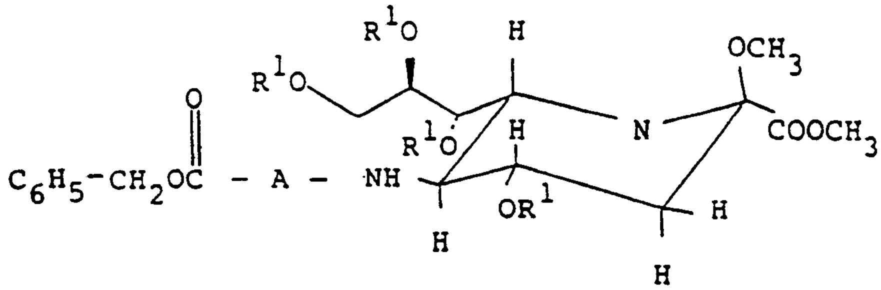

(I-b)

wherein A has the meaning as given in claim 1, and $R^1$ is an acetyl group, which comprises reacting an N-substituted neuraminic acid derivative having the general formula:

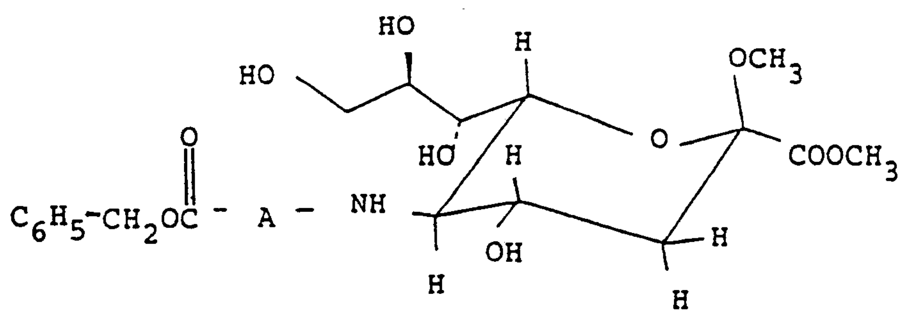

(I-a)

with acetic anhydride in anhydrous pyridine.

5. A process for preparing N-substituted neuraminic acid derivatives having the general formula:

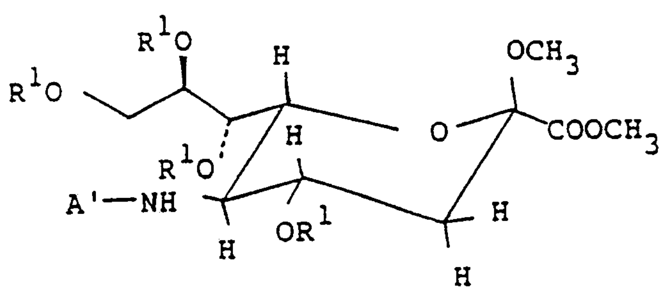

(II-a)

and inorganic and organic acid addition salts thereof, wherein $R^1$ is a hydrogen atom or an acetyl group and A' is an amino acid residue having the general formula:

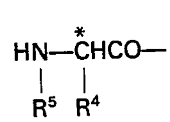

wherein $R^4$ is selected from

$-CH_3$, 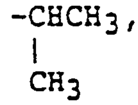, 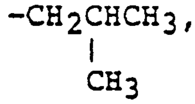,

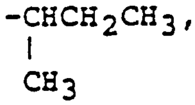, $-CH_2CH_2SCH_3$, $-CH_2C_6H_5$,

$-CH_2OH$, 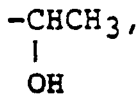, $-CH_2SH$, 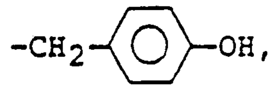,

$-CH_2CONH_2$, $-CH_2CH_2CONH_2$, $-(CH_2)_3NH_2$,

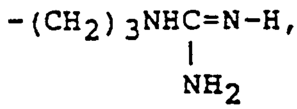, $-CH_2COOH$,

$-CH_2CH_2COOH$, 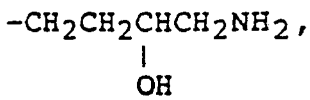,

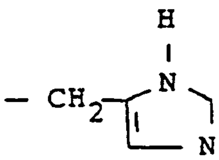 and 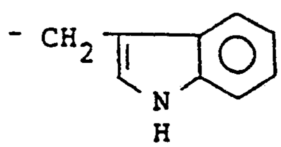

wherein $R^5$ is a hydrogen atom or $R^4$ and $R^5$ form the ring selected from

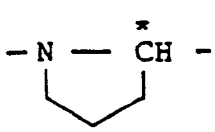 and 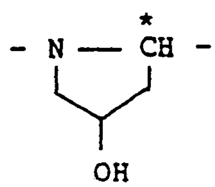

together with the carbon and nitrogen atom to which they are attached, which comprises subjecting an N-substituted neuraminic acid derivative of the general formula:

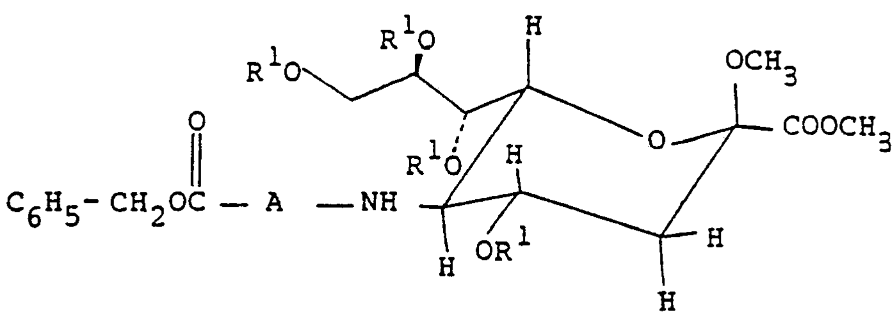

(I)

wherein A has the meaning as given in claim 1, to catalytic reduction.

6. A process as claimed in claim 5 wherein A′ is an amino acid residue having the general formula:

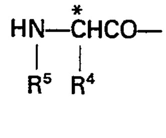

wherein $R^4$ is selected from

$-CH_3$, 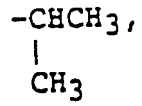 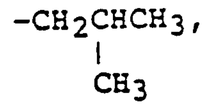

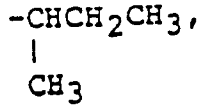 $-CH_2CH_2SCH_3$, $-CH_2C_6H_5$,

$-CH_2OH$, 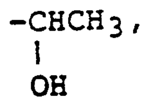 $-CH_2SH$, 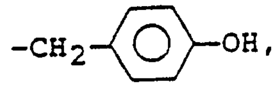

$-CH_2CONH_2$, $-CH_2CH_2CONH_2$, $-(CH_2)_3NH_2$,

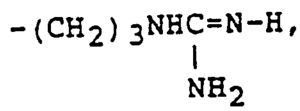 $-CH_2COOH$,

$-CH_2CH_2COOH$, 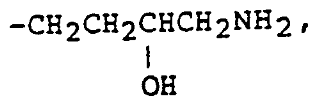

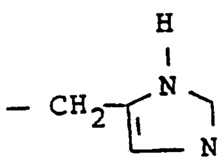 and 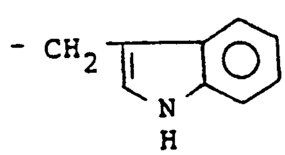

$R^5$ is hydrogen or $R^4$ and $R^5$ form the ring selected from

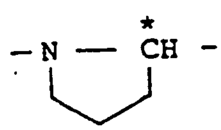 and 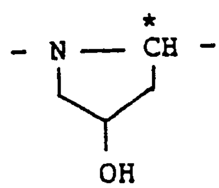

together with the carbon and nitrogen atom to which they are attached.

7. A process as set forth in claim 5 wherein the catalytic reduction is carried out with hydrogen in the presence of a palladium-on-carbon catalyst.

8. Pharmaceutical compositions having immunoregulating effects comprising a compound prepared according to any one of claims 1 to 7.

**Patentansprüche für die Vertragsstaaten: BE CH DE FR GB IT LI LU NL SE**

1. N-substituierte Neuraminsäurederivate der allgemeinen Formel

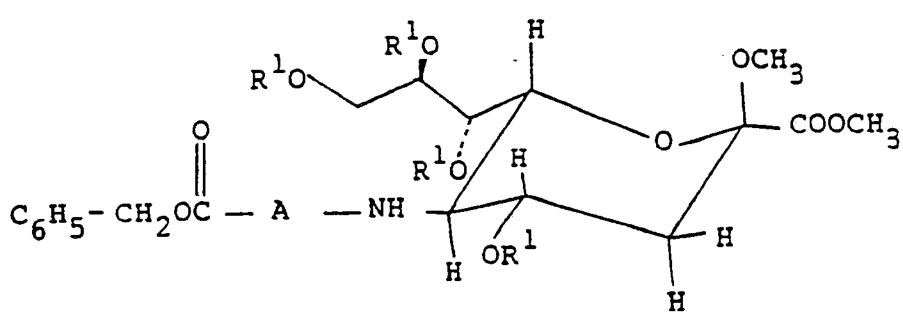 (I)

in der $R^1$ ein Wasserstoffatom oder einen Acetylrest bedeutet und A eine Gruppe der allgemeinen Formel

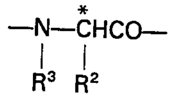

ist, in der $R^2$ ausgewählt ist aus

$-CH_3$, 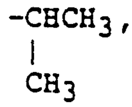 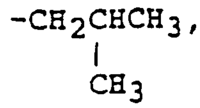

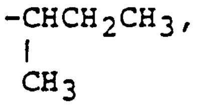 $-CH_2CH_2SCH_3$, $-CH_2C_6H_5$,

$-CH_2OH$, 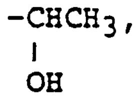 $-CH_2SH$, 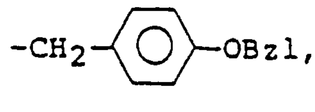

$-CH_2CONH_2$, $-CH_2CH_2CONH_2$. $-(CH_2)_3NH-CBZ$,

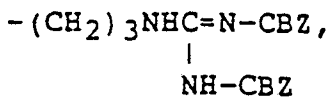 $-CH_2COOBzl$,

$-CH_2CH_2COOBzl$, 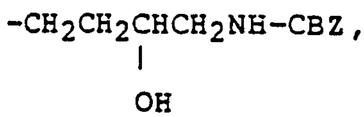

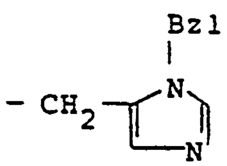 und 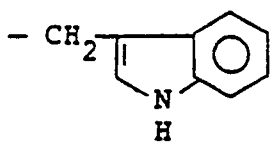

wobei Bzl $CH_2C_6H_5$ ist und CBZ

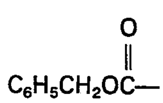 bedeutet;

und $R^3$ ein Wasserstoffatom ist oder $R^2$ und $R^3$ zusammen mit dem Kohlenstoff- und Stickstoffatom, an das sie gebunden sind, einen der Ringe

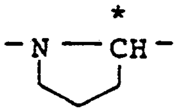 und 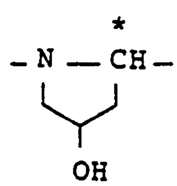

bilden.

2. Verfahren zur Herstellung von N-substituierten Neuraminsäurederivaten der allgemeinen Formel:

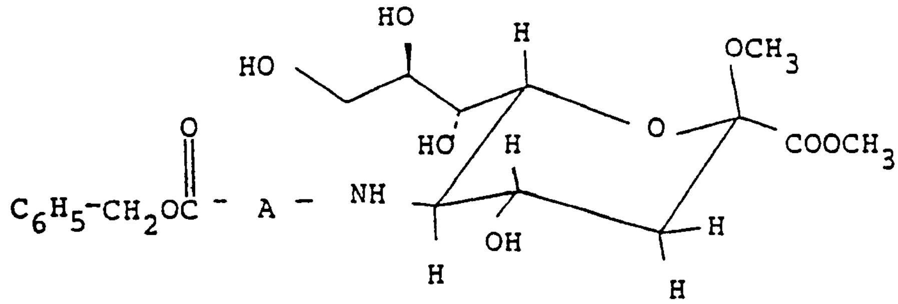

(I-a)

in der A die in Anspruch 1 angegebene Bedeutung hat, das Umsetzung eines N-Benzyloxycarbonylaminosäurederivates der allgemeinen Formel

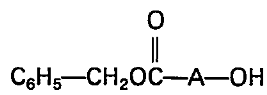

mit einem Neuraminsäurederivat der Formel

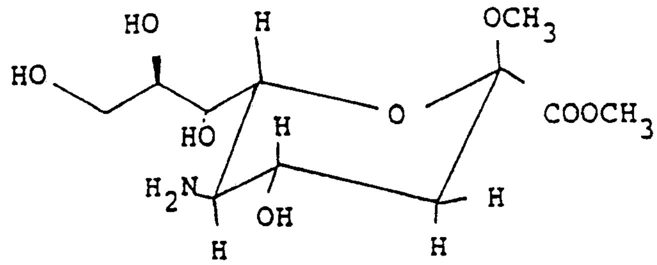

(II)

umfaßt.

3. Verfahren nach Anspruch 2, wobei die Umsetzung in Gegenwart von Dicyclohexylcarbodiimid in einem Lösungsmittel ausgeführt wird, das aus der Gruppe ausgewählt ist, die aus wasserfreiem Pyridin und Dimethylformamid besteht.

4. Verfahren nach Anspruch 2, wobei die N-Hydroxyverbindung zu dem Dimethylformamid in Gegenwart von Dicyclohexylcarbodiimid zugegeben wird.

5. Verfahren zur Herstellung von N-substituierten Neuraminsäurederivaten der allgemeinen Formel

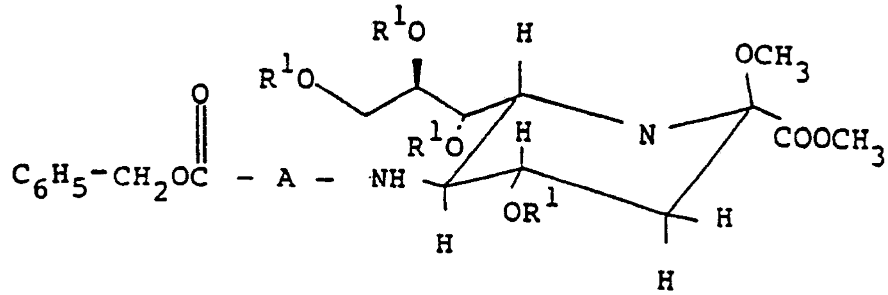

(I-b)

in der A die in Anspruch 1 angegebene Bedeutung hat und R[1] eine Acetylgruppe ist, das Umsetzung eines N-substituierten Neuraminsaürederivates der allgemeinen Formel

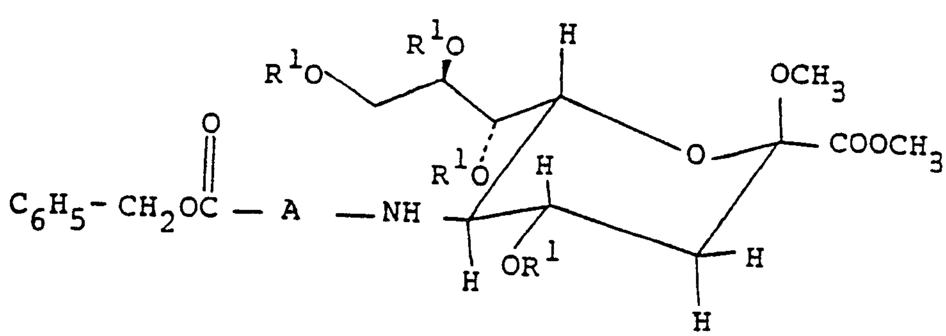

(I)

mit Essigsäureanhydrid in wasserfreiem Pyridin umfaßt.

6. N-substituierte Neuraminsäurederivate der allgemeinen Formel

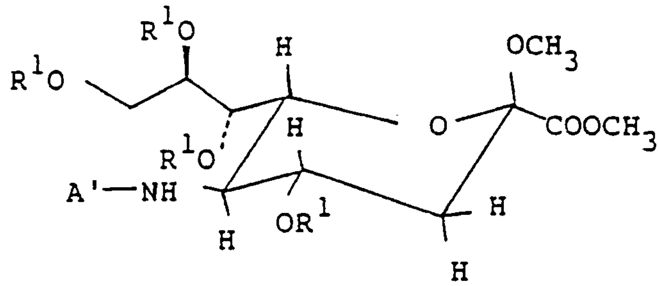

(II-a)

und Additionssalze davon mit anorganischen und organischen Säuren, wobei $R^1$ ein Wasserstoffatom oder einen Acetylrest bedeutet und A′ einen Aminosäurerest der allgemeinen Formel

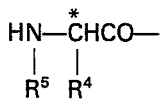

darstellt, wobei $R^4$ ausgewählt ist aus

$-CH_3$, 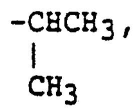 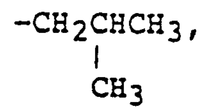

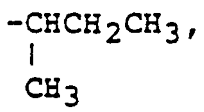 $-CH_2CH_2SCH_3$, $-CH_2C_6H_5$,

$-CH_2OH$, 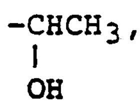 $-CH_2SH$, 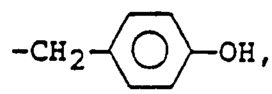

$-CH_2CONH_2$, $-CH_2CH_2CONH_2$, $-(CH_2)_3NH_2$,

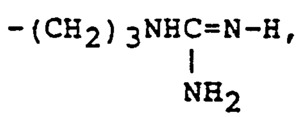 $-CH_2COOH$,

$-CH_2CH_2COOH$, 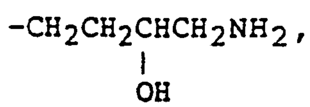

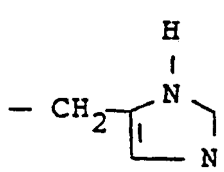 und 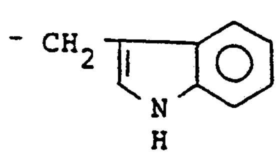

wobei $R^5$ ein Wasserstoffatom ist oder $R^4$ und $R^5$ zusammen mit dem Kohlenstoff- und Stickstoffatom, an das sie gebunden sind, einen der Ringe

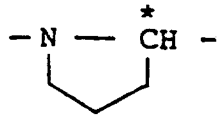 und 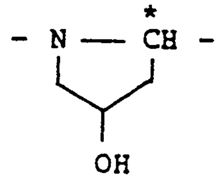

bilden.

7. Verfahren zur Herstellung von N-Neuraminsäurederivaten der allgemeinen Formel

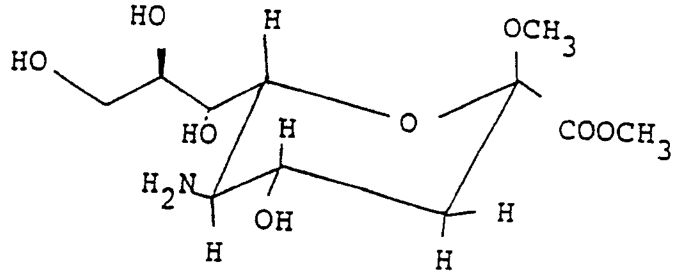

(II)

in der $R^1$ und A' die in Anspruch 6 angegebene Bedeutung haben, und von Additionssalzen davon mit anorganischen und organischen Säuren, das eine katalytische Reduktion eines N-substituierten Neuraminsäurederivates der allgemeinen Formel

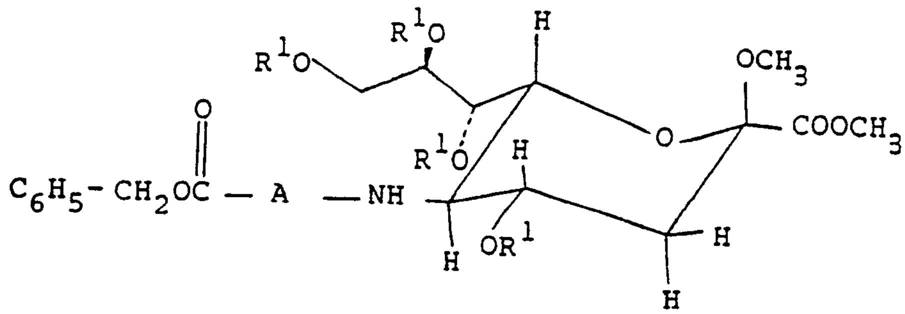

(I)

in der A die in Anspruch 1 angegebene Bedeutung hat, umfaßt.

8. Verfahren nach Anspruch 7, wobei A' ein Aminosäurerest der allgemeinen Formel

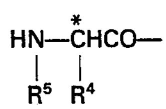

ist, wobei $R^4$ ausgewählt ist aus

$-CH_3$, 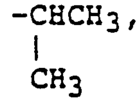 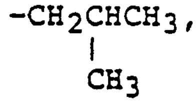

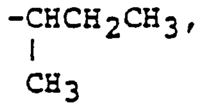 $-CH_2CH_2SCH_3$, $-CH_2C_6H_5$,

$-CH_2OH$, 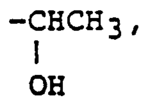 $-CH_2SH$, 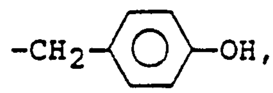

$-CH_2CONH_2$, $-CH_2CH_2CONH_2$, $-(CH_2)_3NH_2$,

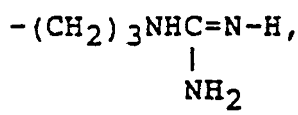 $-CH_2COOH$,

$-CH_2CH_2COOH$, 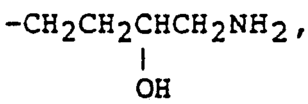

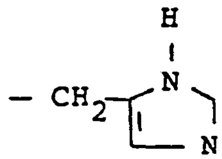 und 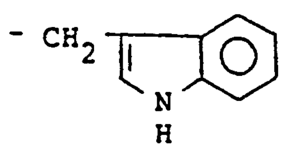

$R^5$ Wasserstoff ist oder $R^4$ und $R^5$ zusammen mit dem Kohlenstoff- und Stickstoffatom, an das sie gebunden sind, einen der Ringe

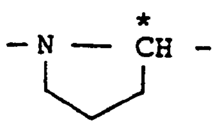 und 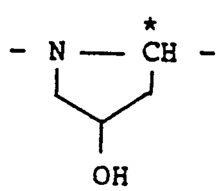

bilden.

9. Verfahren nach Anspruch 7, in dem die katalytische Reduktion mit Wasserstoff in Gegenwart eines Palladium-auf-Kohlenstoff-Katalysators ausgeführt wird.

10. Arzneimittel mit immunregulierenden Wirkungen, umfassend eine Verbindung nach einem der Ansprüche 1 und 6.

**Patentansprüche für den Vertragsstaat: AT**

1. Verfahren zur Herstellung von N-substituierten Neuraminsäurederivaten der allgemeinen Formel

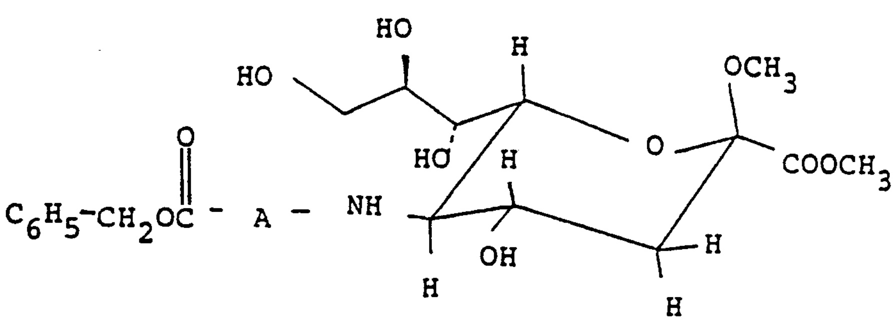

(I-a)

in der A eine Gruppe der folgenden allgemeinen Formel

$$-\underset{R^3}{N}-\underset{R^2}{\overset{*}{C}}HCO-$$

ist, wobei $R^2$ ausgewählt ist aus

$-CH_3$, $-\underset{CH_3}{CH}CH_3$, $-CH_2\underset{CH_3}{CH}CH_3$,

$-\underset{CH_3}{CH}CH_2CH_3$, $-CH_2CH_2SCH_3$, $-CH_2C_6H_5$,

$-CH_2OH$, $-\underset{OH}{CH}CH_3$, $-CH_2SH$, 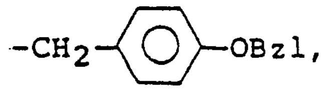,

$-CH_2CONH_2$, $-CH_2CH_2CONH_2$. $-(CH_2)_3NH-CBZ$,

$-(CH_2)_3NH\underset{NH-CBZ}{C}=N-CBZ$, $-CH_2COOBzl$,

$-CH_2CH_2COOBzl$, $-CH_2CH_2\underset{OH}{CH}CH_2NH-CBZ$,

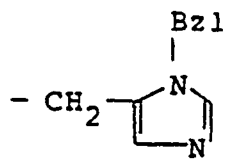 und 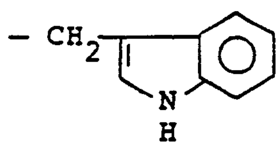

wobei Bzl $-CH_2C_6H_5$ ist und CBZ

$$C_6H_5CH_2O\overset{O}{\overset{\|}{C}}-$$ bedeutet;

und $R^3$ ein Wasserstoffatom ist oder $R^2$ und $R^3$ zusammen mit dem Kohlenstoff- und Stickstoffatom, an das sie gebunden sind, einen der Ringe

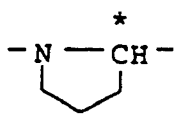 und 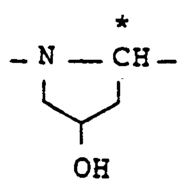

bilden. das die Umsetzung eines N-Benzyloxycarbonylaminosäurederivates der allgemeinen Formel

$$C_6H_5-CH_2O\overset{O}{\overset{\|}{C}}-A-OH$$

mit einem Neuraminsäurederivat der Formel

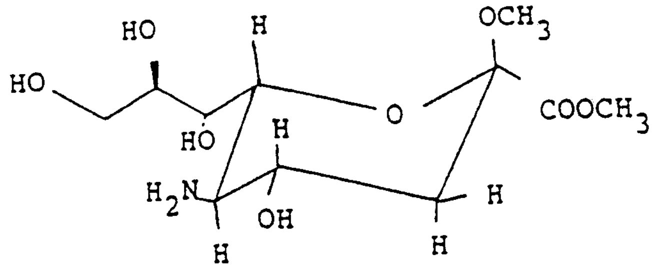

(II)

umfaßt.

2. Verfahren nach Anspruch 1, wobei die Umsetzung in Gegenwart von Dicyclohexylcarbodiimid in einem Lösungsmittel ausgeführt wird, das aus der Gruppe ausgewählt ist, die aus wasserfreiem Pyridin und Dimethylformamid besteht.

3. Verfahren nach Anspruch 1, wobei die N-Hydroxyverbindung zu dem Dimethylformamid in Gegenwart von Dicyclohexylcarbodiimid zugegeben wird.

4. Verfahren zur Herstellung von N-substituierten Neuraminsäurederivaten der allgemeinen Formel

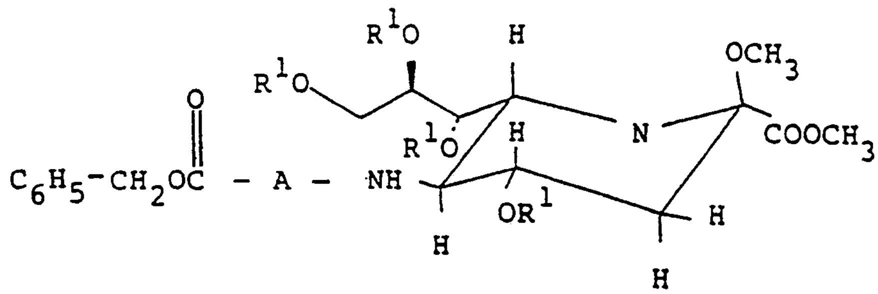

(I-b)

in der A die in Anspruch 1 angegebene Bedeutung hat und $R^1$ eine Acetylgruppe ist, das Umsetzung eines N-substituierten Neuraminsaürederivates der allgemeinen Formel

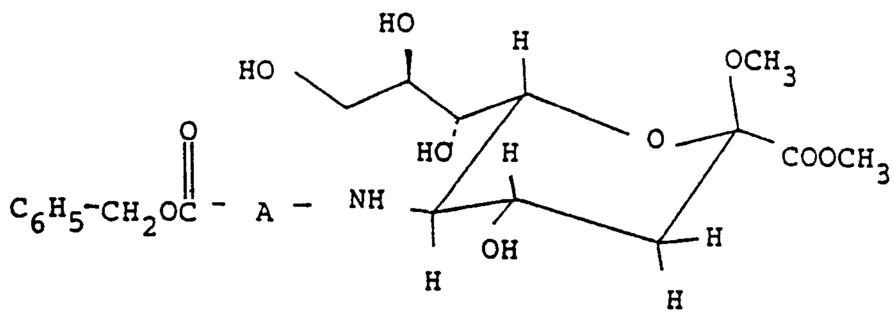

(I-a)

mit Essigsäureanhydrid in wasserfreiem Pyridin umfaßt.

5. Verfahren zur Herstellung von N-substituierte Neuraminsäurederivate der allgemeinen Formel

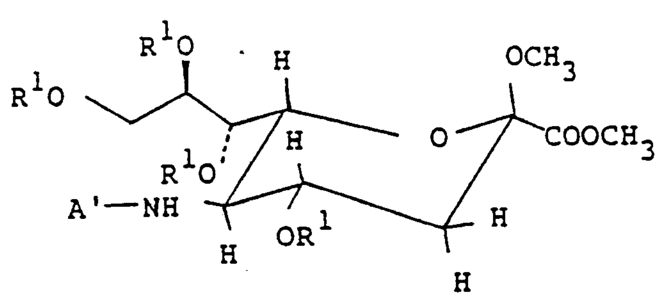

(II-a)

und der Additionssalze davon mit anorganischen un organischen Säuren, wobei $R^1$ ein Wasserstoffatom oder einen Acetylrest bedeutet und A' einen Aminosäurerest der allgemeinen Formel

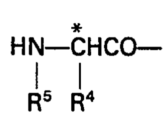

darstellt, wobei $R^4$ ausgewählt ist aus

$-CH_3$, 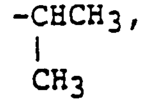 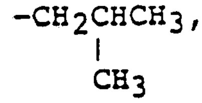

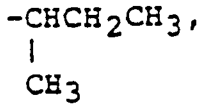 $-CH_2CH_2SCH_3$, $-CH_2C_6H_5$,

$-CH_2OH$, 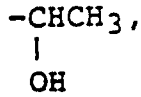 $-CH_2SH$, 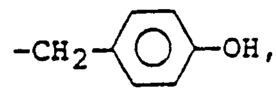

$-CH_2CONH_2$, $-CH_2CH_2CONH_2$, $-(CH_2)_3NH_2$,

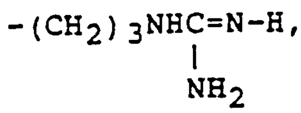 $-CH_2COOH$,

$-CH_2CH_2COOH$, 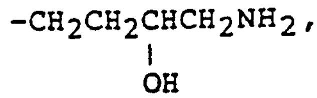

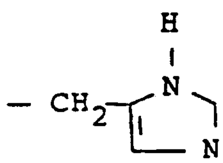 und 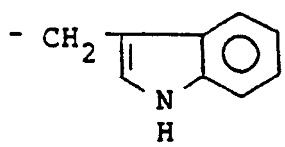

wobei $R^5$ ein Wasserstoffatom ist oder $R^4$ und $R^5$ zusammen mit dem Kohlenstoff- und Stickstoffatom, an das sie gebunden sind, einen der Ringe

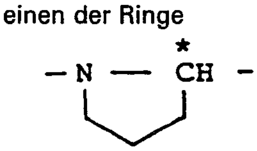 und 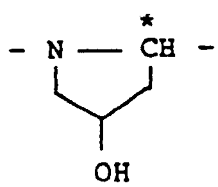

bilden, das eine katalytische Reduktion eines N-substituierten Neuraminsäurederivates der allgemeinen Formel

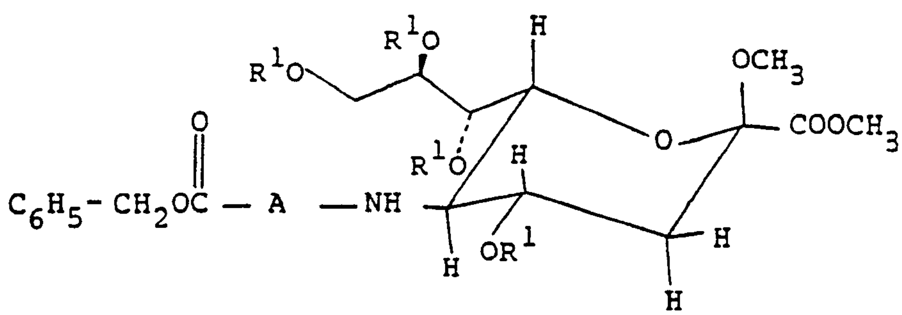 (I)

in der A die in Anspruch 1 angegebene Bedeutung hat, umfaßt.

6. Verfahren nach Anspruch 5, wobei A' ein Aminosäurerest der allgemeinen Formel

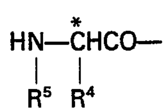

ist, wobei $R^4$ ausgewählt ist aus

$-CH_3$, $-CH(CH_3)CH_3$, $-CH_2CH(CH_3)CH_3$,

$-CH(CH_3)CH_2CH_3$, $-CH_2CH_2SCH_3$, $-CH_2C_6H_5$,

$-CH_2OH$, $-CH(OH)CH_3$, $-CH_2SH$, 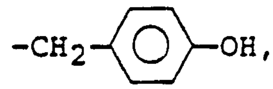

$-CH_2CONH_2$, $-CH_2CH_2CONH_2$, $-(CH_2)_3NH_2$,

$-(CH_2)_3NHC(NH_2)=N-H$, $-CH_2COOH$,

$-CH_2CH_2COOH$, $-CH_2CH_2CH(OH)CH_2NH_2$,

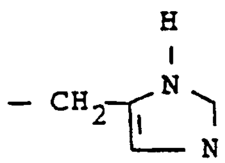 und 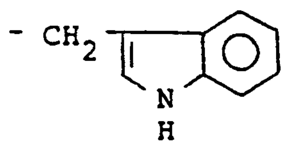

$R^5$ Wasserstoff ist oder $R^4$ und $R^5$ zusammen mit dem Kohlenstoff- und Stickstoffatom, an das sie gebunden sind, einen der Ringe

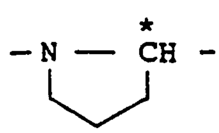 und 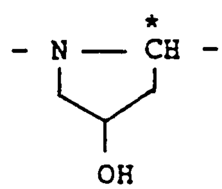

bilden.

7. Verfahren nach Anspruch 5, in dem die katalytische Reduktion mit Wasserstoff in Gegenwart eines Palladium-auf-Kohlenstoff-Katalysators ausgeführt wird.

8. Arzneimittel mit immunregulierenden Wirkungen, umfassend eine Verbindung, hergestellt nach irgendeinem der Ansprüche 1 bis 7.

**Revendications pour les Etats contractants: BE CH DE FR GB IT LI LU NL SE**

1. Dérivés N-substitués de l'acide neuraminique, répondant à la formule générale

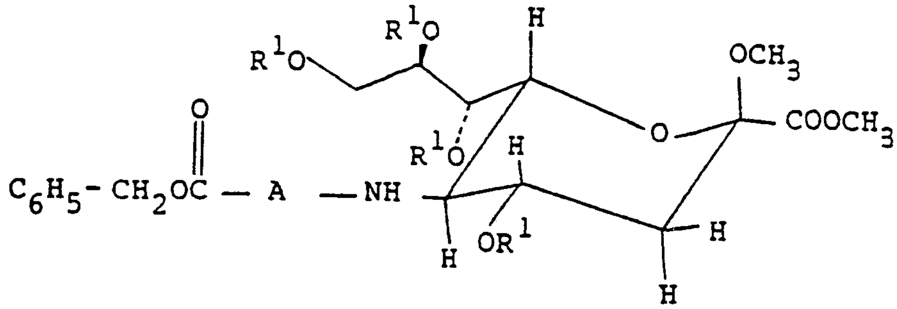

(I)

dans laquelle $R^1$ représente un atome d'hydrogène ou un groupe acétyle, et A représente un groupe de formule générale

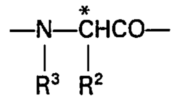

dans laquelle $R^2$ est choisi parmi

$-CH_3$, 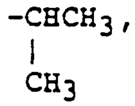 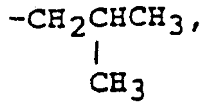

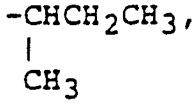 $-CH_2CH_2SCH_3$, $-CH_2C_6H_5$,

$-CH_2OH$, 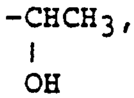 $-CH_2SH$, 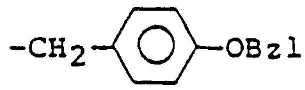

$-CH_2CONH_2$, $-CH_2CH_2CONH_2$. $-(CH_2)_3NH-CBZ$,

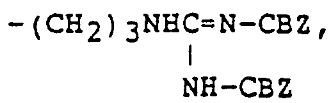 $-CH_2COOBzl$,

$-CH_2CH_2COOBzl$, 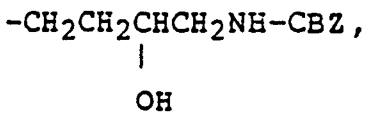

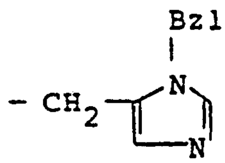 et 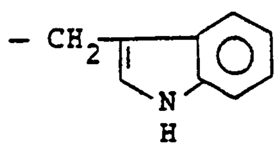

où BZl représente $—CH_2C_6H_5$ et CBZ représente

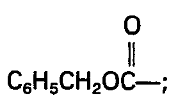

et $R^3$ représente un atome d'hydrogène, ou bien $R^2$ et $R^3$ forment, avec l'atome de carbone et l'atome d'azote auxquels ils sont fixés, un noyau choisi parmi

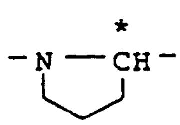 et 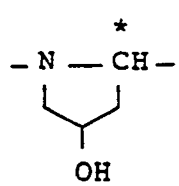

2. Procédé pour préparer des dérivés N-substitués de l'acide neuraminique, répondant à la formule générale:

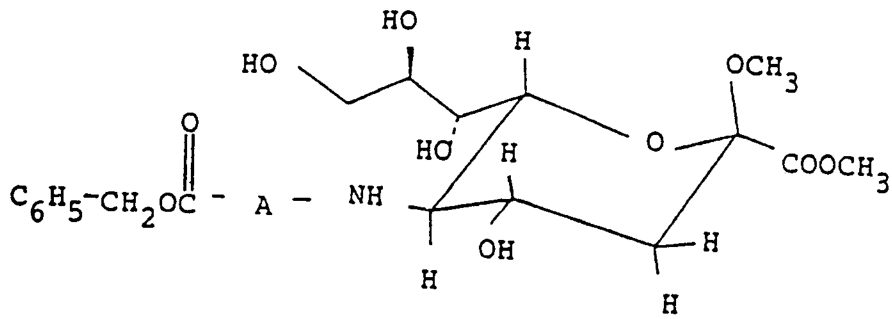

(I-a)

(dans laquelle A a le sens indiqué à la revendication 1), ce procédé comprenant la réaction d'un dérivé de N-benzyloxycarbonylaminoacide répondant à la formule générale:

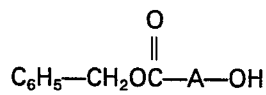

avec un dérivé d'acide neuraminique de formule:

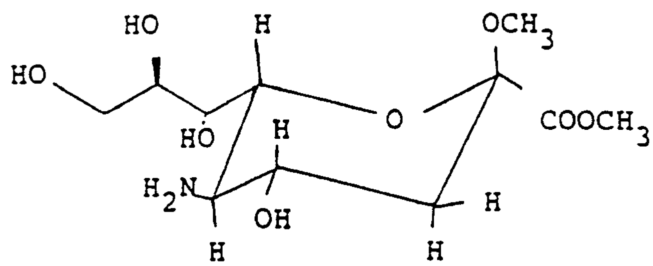

(II)

3. Procédé tel que revendiqué à la revendication 2, dans lequel la réaction est effectuée dans un solvant choisi parmi l'ensemble formé par de la pyridine anhydre et du diméthylformamide, en présence de dicyclohexylcarbodiimide.

4. Procédé tel que revendiqué à la revendication 2, dans lequel on ajoute au diméthylformamide le composé N-hydroxylé, en opérant en présence de dicylohexylcarbodiimide.

5. Procédé pour préparer des dérivés N-substitués de l'acide neuraminique, répondant à la formule générale:

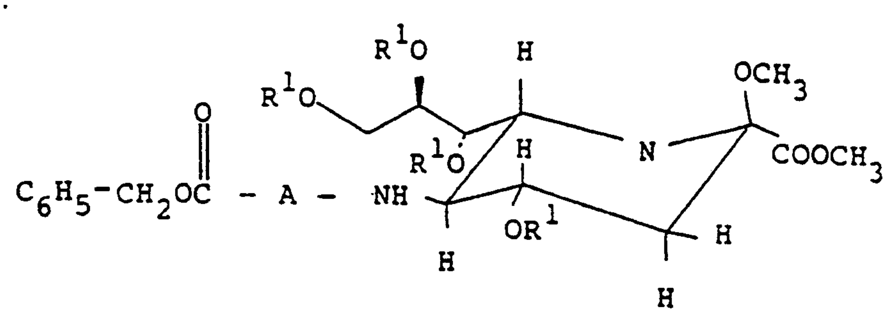

(I-b)

(dans laquelle A a le sens indiqué à la revendication 1, et $R^1$ représente un groupe acétyle), qui comprend la réaction d'un dérivé N-substitué de l'acide neuraminique, répondant à la formule générale:

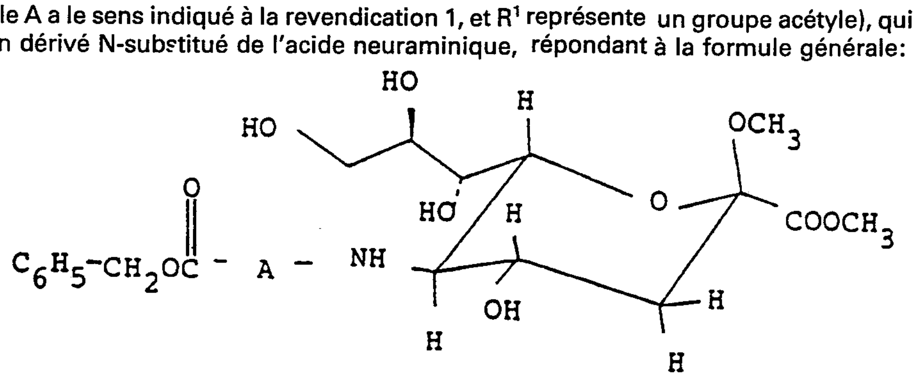

(I-a)

avec l'anhydride acétique dans de la pyridine anhydre.

6. Dérivés N-substitués de l'acide neuraminique, répondant à la formule générale:

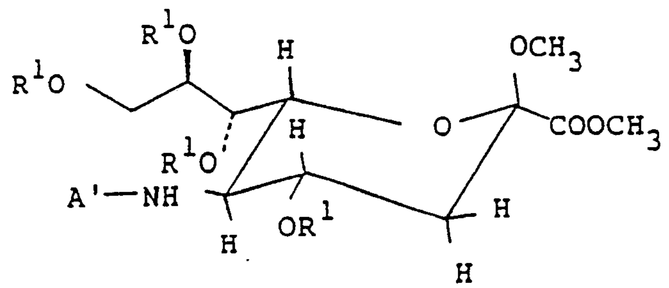

(II-a)

et leurs sels d'addition d'acides minéraux et d'acides organiques, composés dans lesquels $R^1$ représente un atome d'hydrogène ou un groupe acétyle, et A' représente un reste d'aminoacide de formule générale:

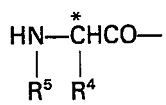

dans laquelle $R^4$ est choisi parmi

$-CH_3$, 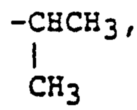, 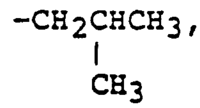,

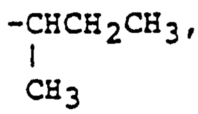, $-CH_2CH_2SCH_3$, $-CH_2C_6H_5$,

$-CH_2OH$, 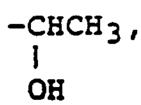, $-CH_2SH$, 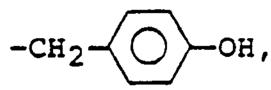,

$-CH_2CONH_2$, $-CH_2CH_2CONH_2$, $-(CH_2)_3NH_2$,

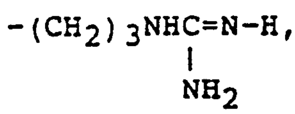, $-CH_2COOH$,

$-CH_2CH_2COOH$, 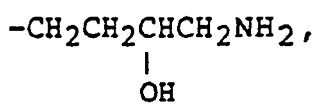,

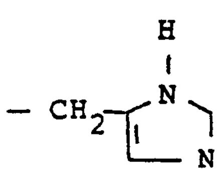 et 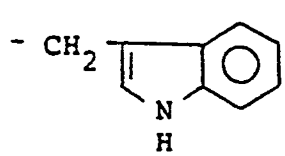

où $R^5$ représente un atome d'hydrogène, ou bien $R^4$ et $R^5$ forment, avec l'atome de carbone et l'atome d'azote auxquels ils sont reliés, un noyau choisi parmi

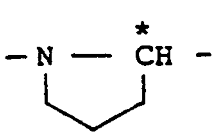 et 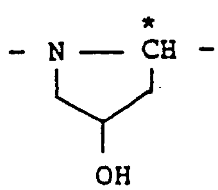

7. Procédé pour préparer des dérivés N-substitués de l'acide neuraminique, répondant à la formule générale:

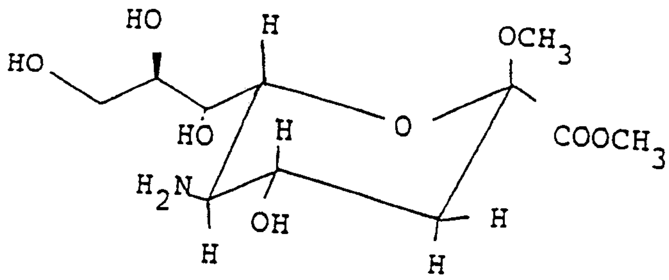

(II)

(dans laquelle R[1] et A' ont le sens indiqué à la revendication 6), et leurs sels d'addition d'acides organiques et d'acides minéraux, qui comprend la soumission d'un dérivé N-substitué de l'acide neuraminique, de formule générale:

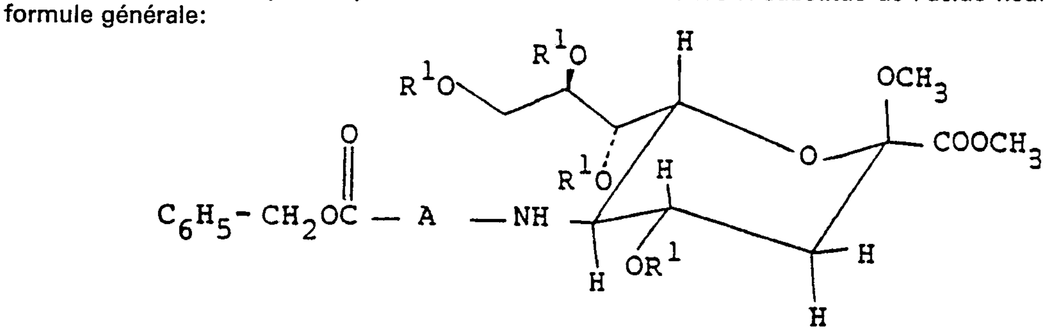

(I)

(dans laquelle A a le sens indiqué à la revendication 1) à une réduction catalytique.

8. Procédé tel que revendiqué à la revendication 7, dans lequel A' représente un reste d'aminoacide de formule:

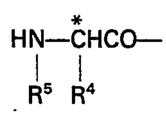

où R[4] est choisi parmi

$-CH_3$, 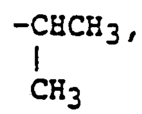 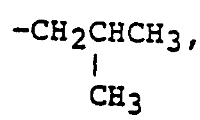

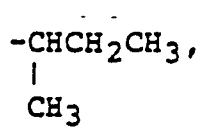 $-CH_2CH_2SCH_3$, $-CH_2C_6H_5$,

$-CH_2OH$, 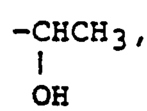 $-CH_2SH$, 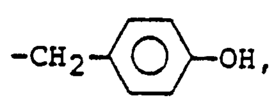

$-CH_2CONH_2$, $-CH_2CH_2CONH_2$, $-(CH_2)_3NH_2$,

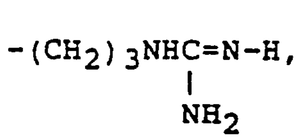 $-CH_2COOH$,

$-CH_2CH_2COOH$, 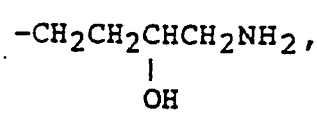

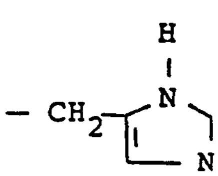 et 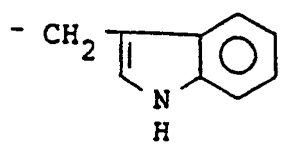

$R^5$ représente un atome d'hydrogène ou bien $R^4$ et $R^5$ forment, avec l'atome de carbone et l'atome d'azote auxquels ils sont fixés, un noyau choisi parmi

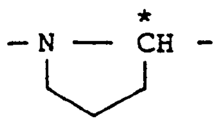 et 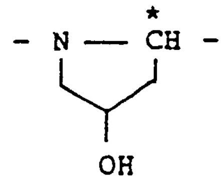

9. Procédé selon la revendication 7, dans lequel on effectue la réduction catalytique avec de l'hydrogène en présence d'un catalyseur à base de palladium sur du charbon.

10. Compositions pharmaceutiques ayant des effets d'immunorégulation, ces compositions comprenant un composé selon l'une des revendications 1 et 6.

**Revendications pour l'Etat contractant: AT**

1. Procédé pour préparer des dérivés N-substitués de l'acide neuraminique, répondant à la formule générale:

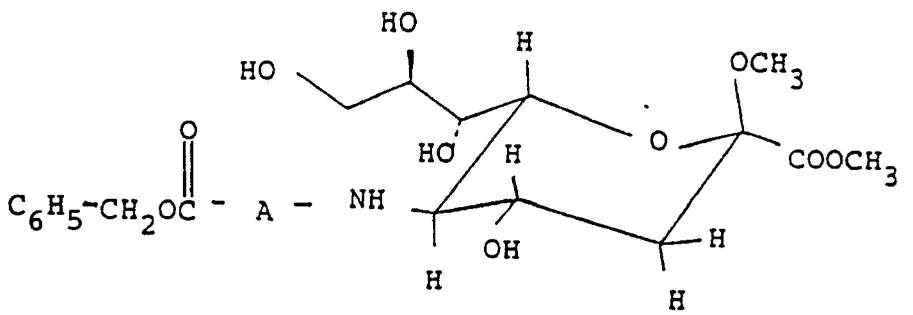

(I-a)

dans laquelle A est un groupe répondant à la formule générale suivante:

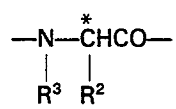

où $R^2$ est choisi parmi

$-CH_3$, 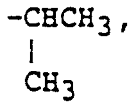 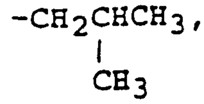

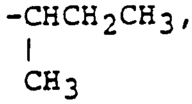 $-CH_2CH_2SCH_3$, $-CH_2C_6H_5$,

$-CH_2OH$, 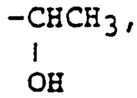 $-CH_2SH$, 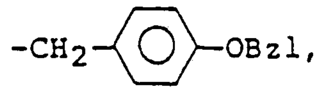

$-CH_2CONH_2$, $-CH_2CH_2CONH_2$. $-(CH_2)_3NH-CBZ$,

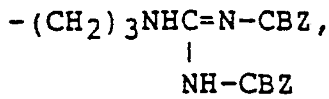 $-CH_2COOBzl$,

$-CH_2CH_2COOBzl$, 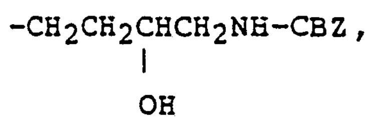

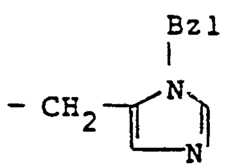 et 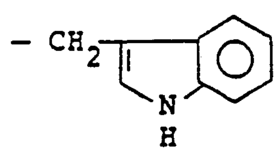

où Bzl représente $—CH_2C_6H_5$ et CBZ représente

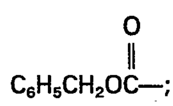

et $R^3$ représente un atome d'hydrogène, ou bien $R^2$ et $R^3$ forment, avec l'atome de carbone et l'atome d'azote auxquels ils sont reliés, un noyau choisi parmi

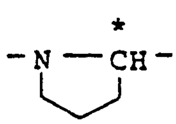 et 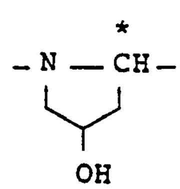

procédé qui comprend la réaction d'un dérivé N-benzyloxycarbonylé d'aminoacide répondant à la formule générale:

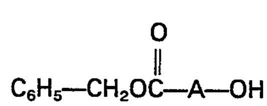

avec un dérivé d'acide neuraminique de formule:

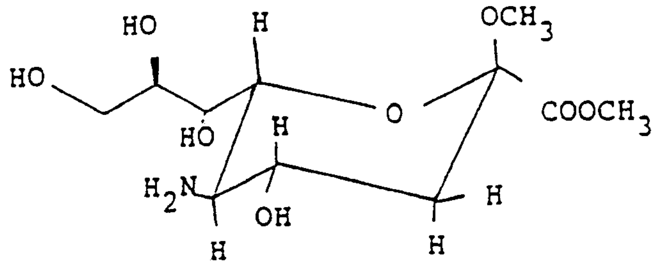

(II)

2. Procédé tel que revendiqué à la revendication 1, dans lequel on effectue la réaction dans un solvant choisi dans l'ensemble constitué par de la pyridine anhydre et du diméthylformamide, en operant en présence de dicyclohexylcarbodiimide.

3. Procédé tel que revendiqué à la revendication 1, dans lequel on ajoute le composé N-hydroxylé au diméthylformamide en présence de dicylohexylcarbodiimide.

4. Procédé pour préparer des dérivés N-substitués de l'acide neuraminique, répondant à la formule générale:

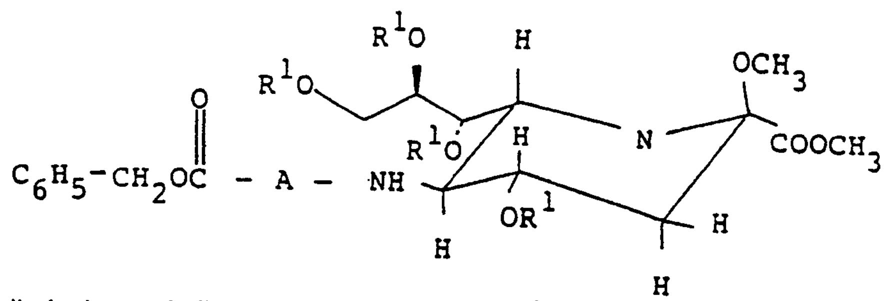

(I-b)

(dans laquelle A a le sens indiqué à la revendication 1, et $R^1$ représente un groupe acétyle), qui comprend la réaction d'un dérivé N-substitué d'acide neuraminique, répondant à la formule générale:

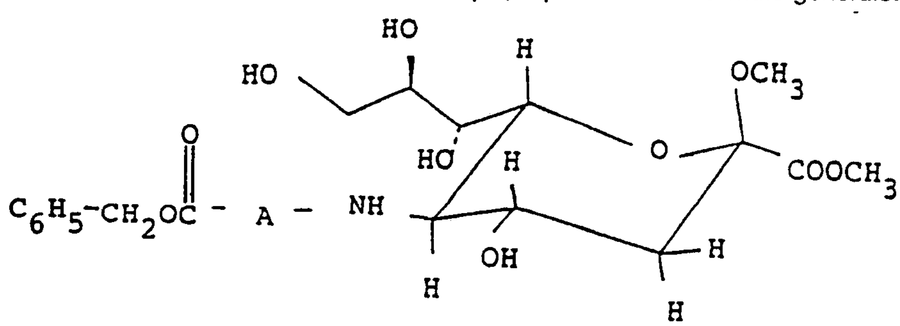

(I-a)

avec l'anhydride acétique dans de la pyridine anhydre.

5. Procédé pour préparer des dérivés N-substitués de l'acide neuraminique, répondant à la formule générale:

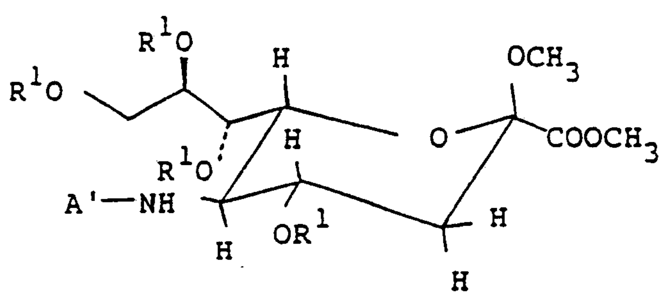

(II-a)

et leurs sels d'addition d'acides minéraux et organiques, composés dans lesquels $R^1$ représente un atome d'hydrogène ou un groupe acétyle, et A' représente un reste d'aminoacide de formule générale:

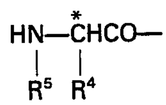

où $R^4$ est choisi parmi

$-CH_3$, 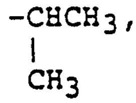 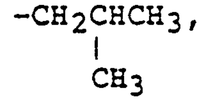

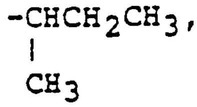 $-CH_2CH_2SCH_3$, $-CH_2C_6H_5$,

$-CH_2OH$, 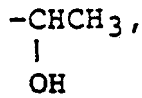 $-CH_2SH$, 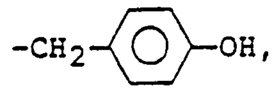

$-CH_2CONH_2$, $-CH_2CH_2CONH_2$, $-(CH_2)_3NH_2$,

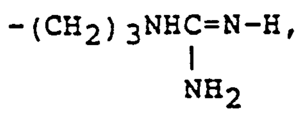 $-CH_2COOH$,

$-CH_2CH_2COOH$, 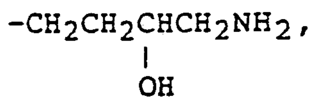

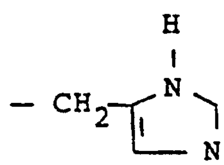 et 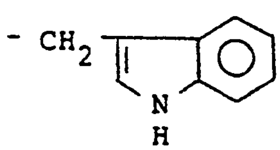

$R^5$ représente un atome d'hydrogène, ou bien $R^4$ et $R^5$ forment, avec l'atome de carbone et l'atome d'azote auxquels ils sont reliés, un noyau choisi parmi

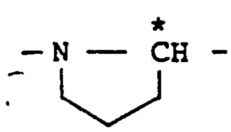 et 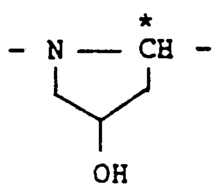

procédé qui comprend la soumission d'un dérivé N-substitué de l'acide neuraminique, répondant à la formule générale:

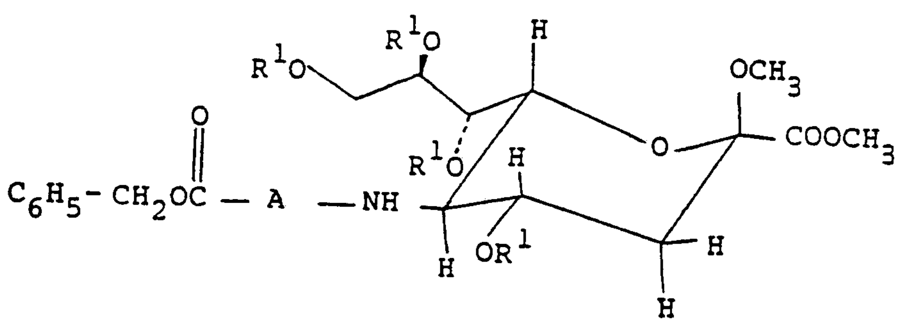 (I)

(dans laquelle A a le sens indiqué à la revendication 1) à une réduction catalytique.

6. Procédé tel que revendiqué à la revendication 5, dans lequel A' représente un reste d'aminoacide répondant à la formule générale:

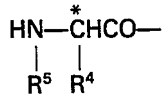

où $R^4$ est choisi parmi

$-CH_3$, 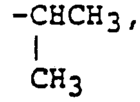 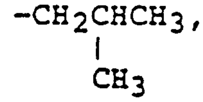

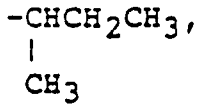 $-CH_2CH_2SCH_3$, $-CH_2C_6H_5$,

$-CH_2OH$, 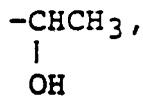 $-CH_2SH$, 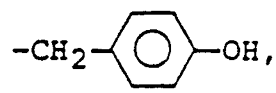

$-CH_2CONH_2$, $-CH_2CH_2CONH_2$, $-(CH_2)_3NH_2$,

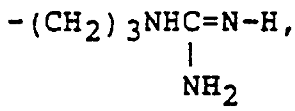 $-CH_2COOH$,

$-CH_2CH_2COOH$, 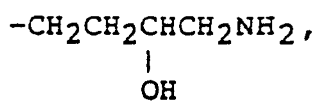

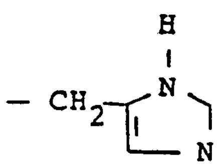 et 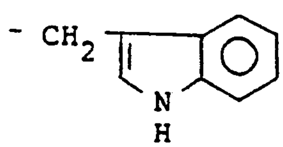

$R^5$ représente un atome d'hydrogène ou bien $R^4$ et $R^5$ forment, avec l'atome de carbone et l'atome d'azote auxquels ils sont reliés, un noyau choisi parmi

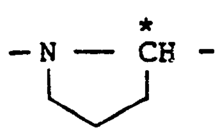 et 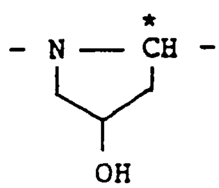

7. Procédé tel que revendiqué à la revendication 5, dans lequel on effectue la réduction catalytique avec de l'hydrogène en présence d'un catalyseur à base de palladium sur du carbone.

8. Compositions pharmaceutiques ayant des effets immunorégulateurs, comprenant un composé préparé selon l'une quelconque des revendications 1 à 7.